# EUROPÄISCHE PATENTSCHRIFT

(11) **EP 1 494 995 B1**
(45) Veröffentlichungstag und Bekanntmachung des Hinweises auf die Patenterteilung: **22.09.2010**
(21) Anmeldenummer: 03702396.7
(22) Anmeldetag: 08.01.2003
(51) Int. Cl.: C07C 215/76, C07C 215/80, C07C 217/80, C07C 223/06, C07D 213/38, C07D 307/81, C07D 317/58, C07D 319/18, A61Q 5/10

(54) **IN 2-STELLUNG SUBSTITUIERTE 3-AMINOPHENOL-DERIVATE SOWIE DIESE VERBINDUNGEN ENTHALTENDE FÄRBEMITTEL**
3-AMINOPHENOL DERIVATIVES SUBSTITUTED IN THE 2-POSITION, AND DYES CONTAINING THESE COMPOUNDS
DERIVES DE 3-AMINOPHENOL SUBSTITUES EN POSITION 2 ET COLORANTS CONTENANT LESDITS COMPOSES

(30) Priorität: 18.04.2002 DE 10217270
(43) Veröffentlichungstag der Anmeldung: 12.01.2005
(73) Patentinhaber: The Procter and Gamble Company, Cincinnati, Ohio 45202 (US)
(72) Erfinder: UMBRICHT, Gisela, CH-1723 Marly (CH); ROSATO, Franco, Jose, CH-3097 Liebefeld (CH); BRAUN, Hans-Jürgen, CH-3182 Überstorf (CH)
(74) Vertreter: Hirsch, Uwe Thomas M.H.
(86) Internationale Anmeldenummer: PCT/EP2003/000097
(87) Internationale Veröffentlichungsnummer: WO 2003/087034

(56) Entgegenhaltungen:
- WO-A-02/02507
- WO-A-97/30968
- DE-A- 4 017 516
- US-A- 5 183 941

## Beschreibung

Die vorliegende Erfindung betrifft neue in 2-Stellung substituierte 3-Aminophenol-Derivate sowie diese Verbindungen enthaltende Mittel zur oxidativen Färbung von Keratinfasern, insbesondere menschlichen Haaren.

Auf dem Gebiet der Färbung von Keratinfasern, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenol, 1,4-Diaminobenzol und 4,5-Diamino-1-(2-hydroxyethyl)-pyrazol eingesetzt, während als Kupplersubstanzen beispielsweise Resorcin, 2-Methyl-resorcin, 1-Naphthol, 3-Aminophenol, 5-Amino-2-methylphenol, m-Phenylendiamin, 2-Amino-4-(2'-hydroxyethyl)amino-anisol, 1,3-Diamino-4-(2'-hydroxyethoxy)benzol und 2,4-Diamino-5-fluor-toluol zu nennen sind.

An Oxidationsfarbstoffe, die zur Färbung menschlicher Haare verwendet werden, werden neben der Färbung in der gewünschten Intensität zahlreiche zusätzliche Anforderungen gestellt. So müssen die Farbstoffe in toxikologischer und dermatologischer Hinsicht unbedenklich sein und die erzielten Haarfärbungen eine gute Lichtechtheit, Dauerwellechtheit, Säureechtheit und Reibechtheit aufweisen. Auf jeden Fall aber müssen solche Färbungen ohne Einwirkung von Licht, Reibung und chemischen Mitteln über einen Zeitraum von mindestens 4 bis 6 Wochen stabil bleiben. Außerdem ist es erforderlich, dass durch Kombination geeigneter Entwicklersubstanzen und Kupplersubstanzen eine breite Palette verschiedener Farbnuancen erzeugt werden kann.

Obwohl bereits eine Vielzahl von Kupplersubstanzen bekannt ist (WO 97/30 968; DE 4 017 516; US 5 183 941), ist es mit den derzeit bekannten Färbemitteln nicht möglich, die an ein Färbemittel gestellten Anforderungen in jeder Hinsicht zu erfüllen. Es besteht daher weiterhin ein Bedürfnis nach neuen Kupplersubstanzen, welche die vorgenannten Anforderung in besonderem Masse erfüllen.

Es wurde nunmehr gefunden, dass bestimmte 3-Aminophenol-Derivate gemäß der allgemeinen Formel (I) die an Kupplersubstanzen gestellten Anforderungen in besonders hohem Masse erfüllen und mit bekannten Entwicklersubstanzen farbstarke, außerordentlich lichtechte und waschechte Farbnuancen ergeben.

Gegenstand der vorliegenden Erfindung sind daher neue 3-Aminophenol-Derivate der vorstehenden Formel (I), oder deren physiologisch verträglichen wasserlösliche Salze, 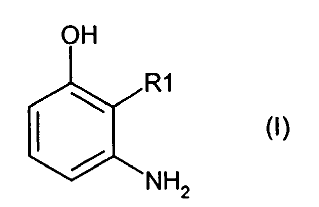 worin **R1** gleich einem Rest der Formel (II) oder (III) ist; 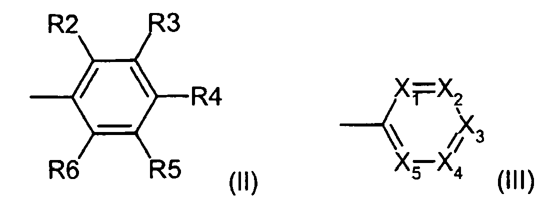 wobei **R2, R3, R4, R5** und **R6** unabhängig voneinander Wasserstoff, ein Halogenatom (F, Cl, Br, J), eine Cyanogruppe, eine Hydroxygruppe, eine C₁-C₄-Alkoxygruppe, eine Phenoxygruppe, eine C₂-C₄-Hydroxyalkoxygruppe, eine C₁-C₆-Alkylgruppe, eine Phenylgruppe, eine C₁-C₄-Alkylthioethergruppe, eine Mercaptogruppe, eine Nitrogruppe, eine Aminogruppe, eine C₁-C₄-Alkylaminogruppe, eine Hydroxy(C₂-C₄)alkylaminogruppe, eine Di(C₁-C₄)alkylaminogruppe, eine Di(hydroxy(C₂-C₄)alkyl)-aminogruppe, eine (Dihydroxy(C₃-C₄)alkyl)-aminogruppe, eine (Hydroxy(C₂-C₄)alkyl)-C₁-C₄-alkylaminogruppe, eine Trifluoromethangruppe, eine Formylgruppe, eine Acetylgruppe, eine Trifluoroacetylgruppe, eine Trimethylsilylgruppe, eine (C₁-C₄)-Hydroxyalkylgruppe, eine (C₂-C₄)-Dihydroxyalkylgruppe, eine (C₁-C₄)-Aminoalkylgruppe, oder eine (C₁-C₄)-Cyanoalkylgruppe darstellen, oder zwei nebeneinanderliegende Reste R2 bis R6 jeweils zusammen mit dem Restmolekül einen heterozyklischen oder carbozyklischen, substituierten oder unsubstituierten Ring bilden;
**X₁**, **X₂**, **X₃**, **X₄** und **X₅** unabhängig voneinander gleich Stickstoff oder einer C-R7-Gruppe, C-R8-Gruppe, C-R9-Gruppe, C-R10-Gruppe oder C-R11-Gruppe sind, unter der Bedingung, dass mindestens einer und höchstens drei der Reste **X1** bis **X5** Stickstoff bedeuten; und
**R7, R8, R9, R10** und **R11** unabhängig voneinander Wasserstoff, ein Halogenatom (F, Cl, Br, J), eine Cyanogruppe, eine C₁-C₆-Alkylgruppe, eine (C₁-C₄)-Alkylthioethergruppe, eine Mercaptogruppe, eine Nitrogruppe, eine Aminogruppe, eine (C₁-C₄)-Alkylaminogruppe, eine Hydroxy(C₂-C₄)alkylamino-gruppe, eine Di(C₁-C₄)alkylaminogruppe, eine Di(hydroxy-(C₂-C₄)alkyl)-aminogruppe, eine (Dihydroxy(C₃-C₄)alkyl)-aminogruppe, eine (Hydroxy(C₂-C₄)alkyl)-C₁-C₄-alkylaminogruppe, eine Trifluoromethangruppe eine Formylgruppe, eine Acetylgruppe, eine Trifluoroacetylgruppe, eine Trimethylsilylgruppe, eine Carbamoylgruppe, eine (C₁-C₄)-Hydroxyalkylgruppe oder eine (C₂-C₄)-Dihydroxyalkylgruppe darstellen.

Als Verbindungen der Formel (I) können beispielweise genannt werden: 6-Amino-[1,1'-biphenyl]-2-ol, 6-Amino-4'-methyl- [1,1'-biphenyl]-2-ol, 6-Amino-3'-methyl-[1,1'-biphenyl]-2-ol, 6-Amino-2'-methyl-[1,1'-biphenyl]-2-ol, 6-Amino-2',3'-dimethyl-[1,1'-biphenyl]-2-ol, 6-Amino-2',4'-dimethyl-[1,1'-biphenyl]-2-ol, 6-Amino-2',5'-dimethyl-[1,1'-biphenyl]-2-ol, 6-Amino-2',6'-dimethyl-[1,1'-biphenyl]-2-ol, 6-Amino-3',4'-dimethyl-[1,1'-biphenyl]-2-ol, 6-Amino-3',5'-dimethyl-[1,1'-biphenyl]-2-ol, 6-Amino-3',6'-dimethyl-[1,1'-biphenyl]-2-ol, 6-Amino-2',4',5'-trimethyl-[1,1'-biphenyl]-2-ol, 6-Amino-2',4',6'-trimethyl-[1,1'-biphenyl]-2-ol, 6-Amino-2',3',4'-trimethyl-[1,1'-biphenyl]-2-ol, 6-Amino-2',3',5'-trimethyl-[1,1'-biphenyl]-2-ol, 6-Amino-2',3',6'-trimethyl-[1,1'-biphenyl]-2-ol, 6-Amino-4'-chlor-[1,1'-biphenyl]-2-ol, 6-Amino-3'-chlor-[1,1'-biphenyl]-2-ol, 6-Amino-2'-chlor-[1,1'-biphenyl]-2-ol, 6-Amino-4'-fluor-[1,1'-biphenyl]-2-ol, 6-Amino-3'-fluor-[1,1'-biphenyl]-2-ol, 6-Amino-2'-fluor-[1,1'-biphenyl]-2-ol, 6-Amino-4'-brom-[1,1'-biphenyl]-2-ol, 6-Amino-3'-brom-[1,1'-biphenyl]-2-ol, 6-Amino-2'-brom-[1,1'-biphenyl]-2-ol, 6-Amino-3',5'-dichlor-[1,1'-biphenyl]-2-ol, 6-Amino-3',5'-difluor-[1,1'-biphenyl]-2-ol, 6-Amino-3'-brom-5'-methyl-1,1'-biphenyl-2-ol, 6-Amino-4'-(trifluoromethyl)-[1,1'-biphenyl]-2-ol, 6-Amino-3'-(trifluoromethyl)-[1,1'-biphenyl]-2-ol, 6-Amino-2'-(trifluoromethyl)-[1,1'-biphenyl]-2-ol, 6-Amino-4'-nitro-[1,1'-biphenyl]-2-ol, 6-Amino-3'-nitro-[1,1'-biphenyl]-2-ol, 6-Amino-2'-nitro-[1,1'-biphenyl]-2-ol, 6-Amino-5'-methyl-3'-nitro[1,1'-biphenyl]-2-ol, 6-Amino-4'-methyl-3'-nitro[1,1'-biphenyl]-2-ol, 6-Amino-2'-methyl-3'-nitro-[1,1'-biphenyl]-2-ol, 6-Amino-2'-nitro-4'-(trifluoromethyl)[1,1'-biphenyl]-2-ol, 6-Amino-3'-nitro-5'-(trifluoromethyl)[1,1'-biphenyl]-2-ol, 6-Amino-3'-nitro-4'-(trifluoromethyl)[1,1'-biphenyl]-2-ol, 6-Amino-3'-nitro-2'-(trifluoromethyl)-[1,1'-biphenyl]-2-ol, 6'-Amino-2'-hydroxy-[1,1'-biphenyl]-4-carbonitril, 6'-Amino-2'-hydroxy-[1,1'-biphenyl]-3- carbonitril, 6-Amino-4'-methoxy-[1,1'-biphenyl]-2-ol, 6-Amino-3'-methoxy-[1,1'-biphenyl]-2-ol, 6-Amino-2'-methoxy-[1,1'-biphenyl]-2-ol, 6-Amino-4'-ethoxy-[1,1'-biphenyl]-2-ol, 6-Amino-3'-ethoxy-[1,1'-biphenyl]-2-ol, 6-Amino-2'-ethoxy-[1,1'-biphenyl]-2-ol, 6-Amino-3',4'-dimethoxy-[1,1'-biphenyl]-2-ol, 6-Amino-3',5'-dimethoxy-[1,1'-biphenyl]-2-ol, 6-Amino-2',3'-dimethoxy-[1,1'-biphenyl]-2-ol, 6-Amino-2',4'-dimethoxy-[1,1'-biphenyl]-2-ol, 6-Amino-2',5'-dimethoxy-[1,1'-biphenyl]-2-ol, 3-Amino-2-(1,3-benzodioxol-5-yl)-phenol, 6-Amino-4'-methoxy-3'-methyl-[1,1'-biphenyl]-2-ol, 6-Amino-4'-methoxy-2'-nitro-[1,1'-biphenyl]-2-ol, 6-Amino-4'-methoxy-3'-nitro-[1,1'-biphenyl]-2-ol, 6-Amino-4'-phenoxy-[1,1'-biphenyl]-2-ol, 6-Amino-4'-methylthio-[1,1'-biphenyl]-2-ol, 6-Amino-3'-methylthio-[1,1'-biphenyl]-2-ol, 6-Amino-2'-methylthio-[1,1'-biphenyl]-2-ol, 6-Amino-[1,1'-biphenyl]-2,4'-diol, 6-Amino-[1,1'-biphenyl]-2,3'-diol, 6-Amino-[1,1'-biphenyl]-2,2'-diol, 2,2',3'-Trihydroxy-6-amino-[1,1'-biphenyl], 2,2',4'-Trihydroxy-6-amino-[1,1'-biphenyl], 2,2',5'-Trihydroxy-6-amino-[1,1'-biphenyl], 2,2',6'-Trihydroxy-6-amino-[1,1'-biphenyl], 2,3',4'-Trihydroxy-6-amino-[1,1'-biphenyl], 2,3',5'-Trihydroxy-6-amino-[1,1'-biphenyl], 6-Amino-2'-methyl-[1,1'-biphenyl]-2,4'-diol, 2',6-Diamino-[1,1'-biphenyl]-2-ol, 3',6-Diamino-[11'-biphenyl]-2-ol, 4',6-Diamino-[1,1'-biphenyl]-2-ol, 4',6-Diamino-[1,1'-biphenyl]-2,2'-diol, 3',6-Diamino-[1,1'-biphenyl]-2,2'-diol, 3',6-Diamino-[1,1'-biphenyl]-2,4'-diol, 3',6-Diamino-[1,1'-biphenyl]-2,5'-diol, 3',6-Diamino-[1,1'-biphenyl]-2,6'-diol, 2',3',6-Triamino-[1,1'-biphenyl]-2-ol, 2',4',6-Triamino-[1,1'-biphenyl]-2-ol, 2',5',6-Triamino-[1,1'-biphenyl]-2-ol, 2',6,6'-Triamino-[1,1'-biphenyl]-2-ol, 3',4',6-Triamino-[1,1'-biphenyl]-2-ol, 3',5',6-Triamino-[1,1'-biphenyl]-2-ol, 1-(6'-Amino-2'-hydroxy-1,1'-biphenyl-4-yl)ethanon, 6-Amino-1,1',3',1"-terphenyl-2-ol, 6-Amino-1,1':4',1"-terphenyl-2-ol, 6-Amino-4'-(aminomethyl)-1,1'-biphenyl-2-ol, 6-Amino-3'-(aminomethyl)-1,1'-biphenyl-2-ol, 6-Amino-2'-(aminomethyl)-1,1'-biphenyl-2-ol, (6'-Amino-2'-hydroxy-1,1'-biphenyl-4-yl)acetonitril, (6'-Amino-2'-hydroxy-1,1'-biphenyl-3-yl)acetonitril, (6'-Amino-2'-hydroxy-1,1'-biphenyl-2-yl)acetonitril, 6'-Amino-2'-hydroxy-1,1'-biphenyl-2-carbaldehyd, 6'-Amino-2'-hydroxy-1,1'-biphenyl-3-carbaldehyd, 6'-Amino-2'-hydroxy-1,1'-biphenyl-4-carbaldehyd, 3-Amino-2-(1-naphthyl)-phenol, 3-Amino-2-(2-naphthyl)phenol, 3-Amino-2-(2,3-dihydro-benzo[1,4]dioxin-6-yl)-phenol, 3-Amino-2-(2,3-dihydro-benzo[1,4]-dioxin-5-yl)-phenol, 3-Amino-2-(2,3-dihydro-benzofuran-5-yl)-phenol, 3-Amino-2-(2,3-dihydro-benzofuran-4-yl)-phenol, 3-Amino-2-(4-pyridinyl)-phenol, 3-Amino-2-(3-pyridinyl)-phenol, 3-Amino-2-(2-pyridinyl)-phenol, 3-Amino-2-(3-methyl-2-pyridinyl)-phenol, 3-Amino-2-(4-methyl-2-pyridinyl)-phenol, 3-Amino-2-(5-methyl-2-pyridinyl)-phenol, 3-Amino-2-(6-methyl-2-pyridinyl)-phenol, 3-Amino-2-(3-chlor-2-pyridinyl)-phenol, 3-Amino-2-(4-chlor-2-pyridinyl)-phenol, 3-Amino-2-(5-chlor-2-pyridinyl)-phenol, 3-Amino-2-(6-chlor-2-pyridinyl)-phenol, 3-Amino-2-(3-fluor-2-pyridinyl)-phenol, 3-Amino-2-(4-fluor-2-pyridinyl)-phenol, 3-Amino-2-(5-fluor-2-pyridinyl)-phenol, 3-Amino-2-(6-fluor-2-pyridinyl)-phenol, 3-Amino-2-(3-trifluoromethyl-2-pyridinyl)-phenol, 3-Amino-2-(4-trifluoromethyl-2-pyridinyl)-phenol, 3-Amino-2-(5-trifluoromethyl-2-pyridinyl)-phenol, 3-Amino-2-(6-trifluoro-methyl-2-pyridinyl)-phenol, 3-Amino-2-(3-nitro-2-pyridinyl)-phenol, 3-Amino-2-(4-nitro-2-pyridinyl)-phenol, 3-Amino-2-(5-nitro-2-pyridinyl)-phenol, 3-Amino-2-(6-nitro-2-pyridinyl)-phenol, 3-Amino-2-(2-methyl-3-pyridinyl)-phenol, 3-Amino-2-(4-methyl-3-pyridinyl)-phenol, 3-Amino-2-(5-methyl-3-pyridinyl)-phenol, 3-Amino-2-(6-methyl-3-pyridinyl)-phenol, 3-Amino-2-(2-chlor-3-pyridinyl)-phenol, 3-Amino-2-(4-chlor-3-pyridinyl)-phenol, 3-Amino-2-(5-chlor-3-pyridinyl)-phenol, 3-Amino-2-(6-chlor-3-pyridinyl)-phenol, 3-Amino-2-(2-brom-3-pyridinyl)-phenol, 3-Amino-2-(4-brom-3-pyridinyl)-phenol, 3-Amino-2-(5-brom-3-pyridinyl)-phenol, 3-Amino-2-(6-brom-3-pyridinyl)-phenol, 3-Amino-2-(2-nitro-3-pyridinyl)-phenol, 3-Amino-2-(4-nitro-3-pyridinyl)-phenol, 3-Amino-2-(5-nitro-3-pyridinyl)-phenol, 3-Amino-2-(6-nitro-3-pyridinyl)-phenol, 3-Amino-2-(5-pyrimidinyl)-phenol und 3-Amino-2-(4-pyrimidinyl)-phenol sowie deren physiologisch verträglichen wasserlöslichen Salze.

Bevorzugt sind Verbindungen der Formel (I) in denen:
(i) **R1** gleich einem Rest der Formel (II) mit **R2** oder **R6** gleich Wasserstoff ist oder (ii) **R1** gleich einem Rest der Formel (III) mit **X1** und **X5** gleich **C-R7** beziehungsweise **C-R11** ist, wobei **R7** beziehungsweise **R11** gleich Wasserstoff sind.

Besonders bevorzugt sind die folgenden Verbindungen der Formel (I): 6-Amino-3'-methoxy-[1,1'-biphenyl]-2-ol, 6-Amino-[1,1'-biphenyl]-2,4'-diol, 3-Amino-2-(2,3-dihydro-benzo[1,4]dioxin-6-yl)-phenol, 3-Amino-2-(2,3-dihydro-benzofuran-5-yl)-phenol, 3-Amino-2-(3-pyridinyl)-phenol, 3-Amino-2-(4-pyridinyl)-phenol und 3-Amino-2-(5-pyrimidinyl)-phenol sowie deren physiologisch verträglichen wasserlöslichen Salze.

Die Verbindungen der Formel (I) können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Die Herstellung der erfindungsgemäßen Aminophenol-Derivate der Formel (I) kann unter Verwendung von literaturbekannten Syntheseverfahren erfolgen, beispielsweise
**a)** durch eine Tetrakis(triphenylphospin)palladium (0) katalysierte Kupplung eines halogensubstituierten 3-Aminophenol-Derivates der Formel (IV) 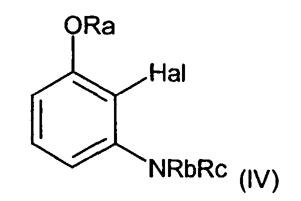 mit einem Borsäurederivat der Formel (IIa) beziehungsweise (IIIa) 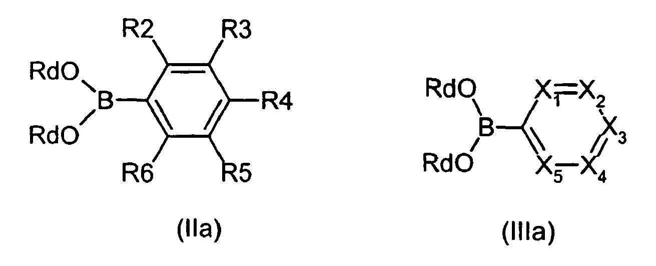 und anschließende Abspaltung der für die Kupplungsreaktion erforderlichen Schutzgruppen und Reduktion einer gegebenenfalls vorhandenen Nitrogruppe; oder
b) durch eine Tetrakis(triphenylphospin)palladium (0) katalysierte Kupplung eines geeigneten substituierten 3-Aminophenol-borsäurederivates der Formel (V) 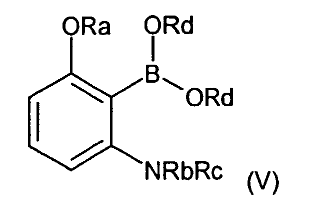 mit einer halogensubstituierten Verbindung der Formel (IIb) beziehungsweise (IIIb) 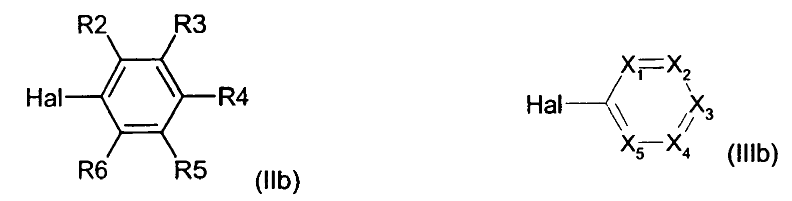 und anschließende Abspaltung der für die Kupplungsreaktion erforderlichen Schutzgruppen und Reduktion einer gegebenenfalls vorhandenen Nitrogruppe; wobei die in den Formeln (IIa), (IIb), (IIIa), (IIIb), (IV) und (V) verwendeten Restgruppen die folgende Bedeutung haben:
   **Ra** steht für eine Schutzgruppe, wie sie zum Beispiel in dem Kapitel "Protective Groups" in Organic Synthesis, Kapitel 3, Wiley Interscience, 1991 beschrieben wird;
   **Rb** und **Rc** stehen unabhängig voneinander für Wasserstoff oder eine Schutzgruppe, wie sie zum Beispiel in dem Kapitel "Protective Groups" in Organic Synthesis, Kapitel 7, Wiley Interscience, 1991 beschrieben wird, oder Rb und Rc bilden gemeinsam mit dem N-Atom eine Nitrogruppe;
   **Rd** ist gleich Wasserstoff oder die beiden Rd-Reste bilden gemeinsam mit der O-B-O-Gruppe einen unsubstituierten oder substituierten fünfgliedrigen oder sechsgliedrigen cycloaliphatischen Ring;
   **Hal** ist gleich F, Cl, Br oder J; und
   **R2, R3, R4, R5** und **R6** sowie **X₁**, **X₂**, **X₃**, **X₄** und **X₅** haben die in der Formel (II) beziehungsweise (III) angegebene Bedeutung.

Die 3-Aminophenol-Derivate der Formel (I) sind gut in Wasser löslich und ermöglichen Färbungen mit ausgezeichneter Farbintensität und Farbechtheit, insbesondere was die Lichtechtheit, Waschechtheit und Reibechtheit anbetrifft. Sie weisen weiterhin eine ausgezeichnete Lagerstabilität, insbesondere als Bestandteil der nachfolgend beschriebenen Oxidationsfärbemittel, auf.

Ein weiterer Gegenstand der vorliegende Erfindung ist daher ein Mittel zur Färbung von Keratinfasern, wie zum Beispiel Wolle, Pelzen, Federn oder Haaren und insbesondere menschlichen Haaren, auf der Basis einer Entwicklersubstanz-Kupplersubstanz-Kombination, welches dadurch gekennzeichnet ist, dass es mindestens ein 3-Aminophenol-Derivat der Formel (I) oder dessen physiologisch verträglichen wasserlösliche Salze enthält.

Die 3-Aminophenol-Derivate der Formel (I) sind in dem erfindungsgemäßen Färbemittel in einer Gesamtmenge von etwa 0,005 bis 20 Gewichtsprozent enthalten, wobei eine Menge von etwa 0,01 bis 5 Gewichtsprozent und insbesondere 0,1 bis 2,5 Gewichtsprozent bevorzugt ist.

Als Entwicklersubstanzen kommen vorzugsweise 1,4-Diamino-benzol (p-Phenylendiamin), 1,4-Diamino-2-methyl-benzol (p-Toluylendiamin), 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 1,4-Diamino-2-(pyridin-3-yl)benzol, 2,5-Diaminobiphenyl, 1,4-Diamino-2-methoxymethyl-benzol, 1,4-Diamino-2-aminomethyl-benzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)-amino]-anilin, 4-[Di(2-hydroxyethyl)amino]-anilin, 4-[Di(2-hydroxyethyl)-amino]-2-methyl-anilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(1-hydroxyethyl)-benzol, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluor-phenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methyl-phenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1H-pyrazol, 4,5-Diamino-1-[(4-methylphenyl)methyl]-1H-pyrazol, 1-[(4-Chlorphenyl)methyl]-4,5-diamino-1H-pyrazol, 4,5-Diamino-1-methyl-1H-pyrazol, 2-Amino-phenol, 2-Amino-6-methyl-phenol, 2-Amino-5-methyl-phenol und 1,2,4-Trihydroxy-benzol in Betracht.

Weiterhin kann das erfindungsgemäße Färbemittel zusätzlich zu den Verbindungen der Formel (I) noch weitere bekannte Kupplersubstanzen, beispielsweise N-(3-Dimethylamino-phenyl)-harnstoff, 2,6-Diamino-pyridin, 2-Amino-4-[(2-hydroxyethyl)amino]-anisol, 2,4-Diamino-1-fluor-5-methylbenzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)amino]-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion, enthalten.

Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemäßen Färbemittel (bezogen auf die Gesamtmenge des Färbemittels) jeweils etwa 0,005 bis 20 Gewichtsprozent, vorzugsweise etwa 0,01 bis 5 Gewichtsprozent und insbesondere 0,1 bis 2,5 Gewichtsprozent, beträgt.

Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6 Gewichtsprozent besonders bevorzugt ist. Die Entwicklersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Überschuß oder Unterschuß vorhanden sind.

Weiterhin kann das erfindungsgemäße Färbemittel zusätzlich andere Farbkomponenten, wie zum Beispiel 6-Amino-2-methylphenol und 2-Amino-5-methylphenol, sowie ferner übliche synthetische oder natürliche direktziehende Farbstoffe, beispielsweise Pflanzenfarbstoffe oder synthetische direktziehende Farbstoffe aus der Gruppe der sauren oder basischen Farbstoffe (beispielsweise die in der WO 95/15144 oder EP-OS 0 850 638 beschriebenen kationischen Farbstoffe), der Triphenylmethanfarbstoffe, der aromatischen Nitrofarbstoffe, der Azofarbstoffe und der Dispersionsfarbstoffe, enthalten. Die erfindungsgemäßen Färbemittel können diese Farbkomponenten in einer Menge von etwa 0,1 bis 4 Gewichtsprozent enthalten.

Selbstverständlich können die zusätzlichen Kupplersubstanzen sowie die Entwicklersubstanzen und die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.

Die Zubereitungsform des erfindungsgemäßen Färbemittels kann beispielsweise eine Lösung, insbesondere eine wässrige oder wässrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie höhere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 30 Gewichtsprozent und die Pflegestoffe in einer Konzentration von etwa 0,1 bis 5 Gewichtsprozent.

Je nach Zusammensetzung kann das erfindungsgemäße Färbemittel schwach sauer, neutral oder alkalisch reagieren. Insbesondere weist es einen pH-Wert von 6,5 bis 11,5 auf, wobei die basische Einstellung vorzugsweise mit Ammoniak erfolgt. Es können aber auch Aminosäuren und/oder organische Amine, zum Beispiel Monoethanolamin und Triethanolamin, sowie anorganische Basen, wie zum Beispiel Natriumhydroxid und Kaliumhydroxid Verwendung finden. Für eine pH-Einstellung im sauren Bereich kommen anorganische oder organische Säuren, zum Beispiel Phosphorsäure, Essigsäure, Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3-bis 12prozentigen, vorzugsweise 6prozentigen, wässrigen Lösung, aber auch Luftsauerstoff in Betracht. Wird eine 6prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugeweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Das erfindungsgemäße Färbemittel mit einem Gehalt an 3-Aminophenol-Derivaten der Formel (I) als Kupplersubstanz ermöglicht Färbungen mit ausgezeichneter Farbechtheit, insbesondere was die Lichtechtheit, Waschechtheit und Reibeechtheit anbetrifft. Hinsichtlich der färberischen Eigenschaften bietet das erfindungsgemäße Färbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt. Hierbei zeichnen sich die Farbtöne durch ihre besondere Farbintensität aus. Die sehr guten färberischen Eigenschaften des Färbemittels gemäß der vorliegenden Anmeldung zeigen sich weiterhin darin, dass dieses Mittel insbesondere auch eine Anfärbung von ergrauten, chemisch nicht vorgeschädigten Haaren problemlos und mit guter Deckkraft ermöglicht.

Die nachfolgenden Beispiele sollen den Gegenstand der Erfindung näher erläutern, ohne ihn darauf zu beschränken.

### Beispiele

### Beispiel 1 bis 17: Synthese von 3-Aminophenol-Derivaten der allgemeinen Formel (I)

### A. Synthese von 2-Brom-3-nitrophenol

Zu einer Suspension von 23,1 g (150 mmol) 2-Amino-3-nitrophenol in 40 ml einer 48%igen Bromwasserstoffsäure und 12 ml Wasser wird bei 0 °C langsam eine Lösung von 10,5 g (152 mmol) Natriumnitrit in 40 ml Wasser zugetropft. Das Gemisch wird anschließend 15 Minuten lang bei 0 °C gerührt. Dann gibt man tropfenweise eine Suspension von 22,5 g Kupfer(I)bromid (Cu₂Br₂; 78,7 mmol) in 75 ml einer 48%igen Bromwasserstoffsäure hinzu und rührt das Gemisch während 15 Minuten bei 0 °C und anschliessend noch 1 Stunde bei 100°C. Anschließend wird das Reaktionsgemisch erneut auf etwa 5 °C gekühlt, filtriert und der Filtrationsrückstand mit wenig Wasser gewaschen. Dieser Rückstand wird sodann in Essigsäureethylester aufgenommen und über Kieselgel filtriert. Anschliessend wird das Lösungsmittel im Vakuum zur Trockenheit eingedampft.
Es werden 32,2 g (98 % der Theorie) 2-Brom-3-nitrophenol erhalten.
Das erhaltene Rohprodukt wird ohne weitere Reinigung in der nächsten Stufe eingesetzt.
¹H-NMR (300 MHz, CDCl₃): δ = 7,47 ppm (dd, J = 1,5 Hz/7,8 Hz, 1H, CH); 7,37 ppm (t, 1H, J = 8,1 Hz, CH); 7,26 ppm (dd, J = 1,5 Hz/8,1 Hz, 1H, CH); 6,08 ppm (s, 1H, OH).
EI-MS: 219/217 [M⁺] (40); 161/159 [M⁺-C-NO₂] (24)

### B. Synthese von 2-Brom-1-(methoxymethoxy)-3-nitrobenzol

15 g (69 mmol) 2-Brom-3-nitrophenol aus Stufe A werden in 150 ml trockenem Acetonitril gelöst und portionenweise mit 3,5 g (117 mmol) einer 80%igen Natriumhydridsuspension bei 0 °C versetzt. Anschliessend wird eine Lösung von 6,1 g (75 mmol) Chlormethoxymethan in 50 ml trockenem Acetonitril zugegeben. Nach beendeter Zugabe wird über Nacht bei Raumtemperatur gerührt. Um überschüssiges Natriumhydrid zu zersetzen werden 10 ml Ethanol zugegeben. Anschließend wird die Reaktionsmischung filtriert und das Filtrat am Rotationsverdampfer im Vakuum zur Trockenheit eingedampft.
Es werden 14,6 g (81 % der Theorie) an 2-Brom-1-(methoxymethoxy)-3-nitrobenzol als braunes Öl erhalten.
Das erhaltene Rohprodukt kann ohne weitere Reinigung in der nächsten Stufe eingesetzt werden.
¹H-NMR (300 MHz, DMSO): δ = 7,60-7,48 ppm (m, 3H, arom.-CH); 5,41 ppm (s, 2H, CH₂); 3,44 ppm (s, 3H, CH₃).
MS (API-ES Neg.): 218/216 [M-H]-(100)

### C. Synthese der 3-Aminophenole der Formel (I)

0,26 g (1 mmol) 2-Brom-1-(methoxymethoxy)-3-nitrobenzol aus Stufe B und 1,5 mmol des entsprechenden Borsäurederivates werden unter Argon in 5 ml 1,2-Dimethoxyethan gelöst. Anschließend werden 0,18 g (0,15 mmol) Tetrakis-(triphenylphosphin)-palladium(0)komplex und 0,8 ml einer wässrigen 2N Kaliumcarbonat-Lösung zugegeben und die Reaktionsmischung auf 100 °C erwärmt. Nach Beendigung der Reaktion wird die Reaktionsmischung in 10 ml Essigsäureethylester gegossen, die organische Phase mit 5 ml Wasser extrahiert, die wässrige Phase noch zweimal mit Essigsäureethylester rückextrahiert, die vereinigten organischen Phasen sodann mit Magnesiumsulfat getrocknet und das Lösungsmittel am Rotationsverdampfer abdestilliert. Der Rückstand wird an Kieselgel mit Heptan/Essigsäureethylester gereinigt.

Das so erhaltene Produkt wird in 5 ml Ethanol gelöst und in Gegenwart von etwa 50 mg Palladium (10 % auf Aktivkohle) bei Raumtempereratur und unter Normaldruck mit Wasserstoffgas hydriert. Nach Beendigung der Reaktion wird das Reaktionsprodukt durch Cellite^{®} filtriert und aufkonzentriert. Der so erhaltene Rückstand wird mit 1 ml einer 2,9molaren ethanolischen Salzsäurelösung oder mit einer 4molaren Salzsäure in Dioxan versetzt. Die Reaktionsmischung wird etwa eine Stunde bei Raumtemperatur gerührt. Nach Beendigung der Reaktion wird der Niederschlag abfiltriert, mit Ethanol (oder Dioxan) gewaschen und sodann getrocknet. Falls keine Ausfällung stattfindet, kann das Lösungsmittel am Rotationsverdampfer abgedampft werden.
1. 6-Amino-1,1'-biphenyl-2-ol-hydrochlorid
   Verwendetes Borsäurederivat: Phenylborsäure
   Ausbeute: 0,088 g (44 % der Theorie)
   ¹H-NMR (300 MHz, DMSO): δ = 9,80 ppm (s, 1H, OH); 7,48 - 7,35 ppm (m, 5H, arom. H); 7,22 ppm (t, J = 7,5 Hz, 1H, arom. H);
   6,89 ppm (t, J = 7,5 Hz, 2H, arom. H); 4,1 - 3,3 ppm (br, 3H, NH3⁺).
   MS (API-ES Pos.): 186 [M+H]⁺ (100)
2. 3',6-Diamino-1,1'-biphenyl-2-ol-dihydrochlorid
   Verwendetes Borsäurederivat: 3-Aminophenylborsäure
   Ausbeute: 0,154 g (34 % der Theorie)
   MS (API-ES Pos.): 201 [M+H]⁺ (60); 223 [M+Na]⁺ (60).
3. 6-Amino-3'-methoxy-1,1'-biphenyl-2-ol-hydrochlorid
   Verwendetes Borsäurederivat: 3-Methoxyphenylborsäure
   Ausbeute: 0,141 g (58 % der Theorie)
   ¹H-NMR (300 MHz, DMSO): δ = 9,80 ppm (s, 1H, OH); 7,38 ppm (t, J=8,1 Hz, 1H, arom. H); 7,22 ppm (t, J = 8,1 Hz, 1H, arom. H); 6,99-6,87 ppm (m, 5H, arom. H); 3,80 ppm (s, 3H, CH₃); 4,1 - 3,3 ppm (br, 3H, NH3⁺).
   MS (API-ES Pos.): 216 [M+H]⁺(100); 238 [M+Na]⁺ (25).
4. 6-Amino-4'-methoxy-1,1'-biphenyl-2-ol-hydrochlorid
   Verwendetes Borsäurederivat: 4-Methoxyphenylborsäure
   Ausbeute: 0,055 g (21 % der Theorie)
   ¹H-NMR (300 MHz, DMSO): δ = 9,70 ppm (s, 1H, OH); 7,27 ppm (d, J=8,6 Hz, 2H, arom.H); 7,18 ppm (t, J=8,1 Hz, 1H, arom. H); 7,02 ppm (d, J=8,6 Hz, 2H, arom. H); 6,84 ppm (t, J = 8,1 Hz, 2H, arom. H); 3,82 ppm (s, 3H, CH₃); 3,7 - 3,3 ppm (br, NH3⁺).
   MS (API-ES Pos.): 216 [M+H]⁺ (100).
5. 3-Amino-2-(1,3-benzodioxol-5-yl)phenol-hydrochlorid
   Verwendetes Borsäurederivat: 3,4-Methylendioxyphenylborsäure Ausbeute: 0,153 g (59 % der Theorie)
   ¹H-NMR (300 MHz, DMSO): δ = 9,81 ppm (s, 1H, OH); 7,20 ppm (t, J=8,1 Hz, 1H, arom. H); 7,01 ppm (d, J=7,9 Hz, 1H, arom. H); 6,92-6,79 ppm (m, 4H, arom. H); 6,80 ppm (s, 2H, CH₂); 3,8 - 3,2 ppm (br, NH₃⁺). MS (API-ES Pos.): 230 [M+H]⁺ (75).
6. 6-Amino-2',4'-dimethoxy-1,1'-biphenyl-2-ol-hydrochlorid
   Verwendetes Borsäurederivat: 2,4-Dimethoxyphenylborsäure
   Ausbeute: 0,078 g (29 % der Theorie)
   MS (API-ES Pos.): 246 [M+H]⁺ (100).
7. 6-Amino-4'-methyl-1,1'-biphenyl-2-ol-hydrochlorid
   Verwendetes Borsäurederivat: 4-Tolylborsäure
   Ausbeute: 0,177 g (78 % der Theorie)
   ¹H-NMR (300 MHz, DMSO): δ = 9,76 ppm (s, 1H, OH); 7,29-7,18 ppm (m, 5H, arom. H); 6,88 ppm (t, J=8,7 Hz, 2H, arom. H); 3,8 - 3,2 ppm (br, NH₃⁺); 2,38 ppm (s, 3H, CH₃).
   MS (API-ES Pos.): 200 [M+H]⁺ (100).
8. 3-Amino-2-(1-naphthyl)phenol-hydrochlorid
   Verwendetes Borsäurederivat: 1-Naphthylborsäure
   Ausbeute: 0,140 g (51 % der Theorie)
   ¹H-NMR (300 MHz, DMSO): δ = 9,66 ppm (s, 1H, OH); 8,01 ppm (d, J=8,1 Hz, 2H, arom. H); 7,65-7,13 ppm (m, 6H, arom. H); 6,90 ppm (d, J=8,1 Hz, 1H, arom. H); 3,8 - 3,2 ppm (br, NH₃⁺).
   MS (API-ES Pos.): 236 [M+H]⁺ (100).
9. 6'-Amino-2'-hydroxy-1,1'-biphenyl-3-carbaldehyd-hydrochlorid
   Verwendetes Borsäurederivat: 3-Formylphenylborsäure
   Ausbeute: 0,104 g (41 % der Theorie)
   MS (API-ES Pos.): 214 [M+H]⁺ (100).
10. 3-Amino-2-(2,3-dihydro-benzo[1,4]dioxin-6-yl)-phenol-hydrochlorid
   Verwendetes Borsäurederivat: 2,3-Dihydro-1,4-benzodioxin-6-ylborsäure
   Ausbeute: 0,05 g (23 % der Theorie)
   MS (API-ES Pos.): 244 [M+H]⁺(100).
11. 3-Amino-2-(2,3-dihydro-benzofuran-5-yl)-phenol-hydrochlorid
   Verwendetes Borsäurederivat: 2,3-Dihydro-1-benzofuran-5-ylborsäure
   Ausbeute: 0,07 g (26 % der Theorie)
   ¹H-NMR (300 MHz, DMSO): δ = 9,62 ppm (s, 1H, OH); 7,17-7,13 ppm (m, 2H, arom. H); ); 7,02 ppm (d, J=7,4 Hz, 1H, arom. H); ); 6,86-6,77 ppm (m, 3H, arom. H); 4,59 ppm (t, J=8,6 Hz, 2H, CH₂); 3,22 ppm (t, J=8,6 Hz, 2H, CH₂); 3,8 - 3,2 ppm (br, NH₃⁺).
   MS (API-ES Pos.): 228 [M+H]⁺(100).
12. 6-Amino-1,1'-biphenyl-2,4'-diol-hydrochlorid
   Verwendetes Borsäurederivat: 4-(Tetrahydro-pyran-2-yloxy)-phenylborsäure
   Ausbeute: 0,03 g (14 % der Theorie)
   MS (API-ES Pos.): 202 [M+H]⁺ (45).
13. 6'-Amino-2'-hydroxy-1,1'-biphenyl-4-carbaldehyd-hydrochlorid
   Verwendetes Borsäurederivat: 4-Formylphenylborsäure
   Ausbeute: 0,07 g (30 % der Theorie)
   MS (API-ES Pos.): 214 [M+H]⁺ (80).
14. 3-Amino-2-(2-trifluoromethylphenyl)-phenol-hydrochlorid
   Verwendetes Borsäurederivat: 2-Trifluoromethylphenylborsäure
   Ausbeute: 0,045 g (15 % der Theorie)
   ¹H-NMR (300 MHz, DMSO): δ = 9,58 ppm (s, 1H, OH); 7,91 ppm (d, J=7,5 Hz, 1H, arom. H); 7,02 ppm (d, J=7,4 Hz, 1H, arom. H); 7,74 ppm (t, J=7,26 Hz, 1H, arom. H); 7,63 ppm (t, J=7,65 Hz, 1H, arom. H); 7,30 ppm (d, J=7,47 ppm, 1H, arom. H); 7,15 ppm (t, J=7,8 Hz, 1H, arom. H); 6,65-6,63 ppm (m, 2H, arom. H); 3,8 - 3,2 ppm (br, NH₃⁺).
   MS (API-ES Pos.): 254 [M+H]⁺ (100).
15. 2',6-Diamino-1,1'-biphenyl-2-ol-dihydrochlorid
   Verwendetes Borsäurederivat: N-(tert.-Butoxycarbonyl)-2-amino-1-phenylborsäure
   Ausbeute: 0,120 g (44 % der Theorie)
   MS (API-ES Pos.): 201 [M+H]⁺ (50); 223 [M+Na] (35).
16. 3-Amino-2-(3-pyridinyl)phenol-hydrochlorid
   Verwendetes Borsäurederivat: 2-(Pyridin-3-yl)-1,3,2-dioxaborolan
   Ausbeute: 0,088 g (39 % der Theorie)
   ¹H-NMR (300 MHz, DMSO): δ = 10,01 ppm (s, 1H, OH); 8,91-8,86 ppm (m, 2H, arom. H); 8,53 ppm (d, J=7,89 Hz, 1H, arom. H); 8,10 ppm (dd, J=5,76 und 7,8 Hz, 1H, arom. H); 7,18 ppm (t, J=8,1 Hz, 1H, arom. H); 6,68-6,65 ppm (m, 2H, arom. H); 4,1 - 3,5 ppm (br, NH₃⁺).
   MS (API-ES Pos.): 187 [M+H]⁺ (100).
17. 3-Amino-2-(4-pyridinyl)phenol-hydrochlorid
   Verwendetes Borsäurederivat: Pyridin-4-ylborsäure
   Ausbeute: 0,130 g (58 % der Theorie)
   ¹H-NMR (300 MHz, DMSO): δ = 9,90 ppm (s, 1H, OH); 8,89 ppm (d, J=6,54 Hz, 2H, arom._{Py} H); 8,05 ppm (d, J=6,54 Hz, 2H, arom._{Py} H); 7,07 ppm (t, J= 7,8 Hz, 1H, arom. H); 6,48-6,42 ppm (m, 2H, arom. H); 4,0 - 3,4 ppm (br, NH₃⁺).
   MS (API-ES Pos.): 187 [M+H]⁺ (100).

### Beispiele 18 bis 34: Haarfärbemittel

Es werden Haarfärbelösungen der folgenden Zusammensetzung hergestellt:

| | |
|---|---|
| 1,25 mmol | Substanz der Formel (I) gemäß Tabelle 1 |
| 1,25 mmol | Entwicklersubstanz gemäß Tabelle 1 |
| 10,0 g | Laurylethersulfat (28prozentige wässrige Lösung) |
| 9,0 g | Ammoniak (22prozentige wässrige Lösung) |
| 7,8 g | Ethanol |
| 0,3 g | Ascorbinsäure |
| 0,3 g | Ethylendiaminotetraessigsäure-Dinatriumsalz-Hydrat |
| ad 100,0 g | Wasser, vollentsalzt |

10 g der vorstehenden Färbelösung werden unmittelbar vor der Anwendung mit 10 g einer 6prozentigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die resultierenden Färbungen sind in Tabelle 1 zusammengefasst.

**Tabelle 1:**

| **Beispiel Nr.** | **Kupplersubstanz der Formel (I)** | **Entwicklersubstanz** | | | | |
|---|---|---|---|---|---|---|
| | | **I.** | **II.** | **III.** | **IV.** | **V.** |
| | | **2,5-Diaminotoluol-sulfat** | **2,5-Diamino-phenylethanol-sulfat** | **4,5-Diamino-1-(2'-hydroxy-ethyl)-pyrazol-sulfat** | **4-Aminophenol** | **2,4,5,6-Tetraamino-pyrimidin-sulfat** |
| **18** | gemäß Beispiel **1** | violett | violett | himbeerrot | schwach orange-braun | blaugrau |
| **19** | gemäß Beispiel **2** | violett | violett | himbeerrot | schwach orange-braun | blaugrau |
| **20** | gemäß Beispiel **3** | dunkel-violett | violett | himbeerrot | orange | grau |
| **21** | gemäß Beispiel **4** | violett | violett | himbeerrot | Schwach orange braun | grau |
| **22** | gemäß Beispiel **5** | violett | violett | himbeerrot | schwach orange-braun | grau |
| **23** | gemäß Beispiel **6** | braun | braun | himbeerrot | schwach orange-braun | schwach grün-braun |
| **24** | gemäß Beispiel **7** | violett | violett | himbeerrot | schwach orange | grau |
| **25** | gemäß Beispiel **8** | violettstichiges Braun | violettstichiges Braun | himbeerrosa | schwach orange | grau |
| **26** | gemäß Beispiel **9** | braunstichiges Violett | braunstichiges Violett | himbeerrot | orange | grau |
| **27** | gemäß Beispiel **10** | violett-braun | violett-braun | rot | schwach orange | grau |
| **28** | gemäß Beispiel **11** | violett | violett | rot | schwach orange | grau |
| **29** | gemäß Beispiel **12** | rotviolett | rotviolett | rot | orange | grau |
| **30** | gemäß Beispiel **13** | violettstichiges Grau | violettstichiges Grau | himbeerrot | schwach orange-braun | grau |
| **31** | gemäß Beispiel **14** | violettstichiges Grau | violettstichiges Grau | himbeerrosa | schwach orange | grünstichiges Grau |
| **32** | gemäß Beispiel **15** | violett | violett | himbeerrot | schwach orange | grau |
| **33** | gemäß Beispiel **16** | intensives Violett | intensives Violett | rot | orange-braun | stahlgrau |
| **34** | gemäß Beispiel **17** | intensives Violett | intensives Violett | rot | orange-braun | mattes Grün |

### Beispiel 35 bis 58: Haarfärbemittel

Es werden Haarfärbelösungen der folgenden Zusammensetzung hergestellt:

| | |
|---|---|
| X g | 3-Aminophenol-Derivat der Formel (I) |
| | (Kupplersubstanz **K1** bis **K4** gemäss Tabelle 4) |
| U g | Entwicklersubstanz **E8** bis **E15** gemäß Tabelle 2 |
| Y g | Kupplersubstanz **K12** bis **K36** gemäß Tabelle 4 |
| 10,0 g | Laurylethersulfat (28prozentige wässrige Lösung) |
| 9,0 g | Ammoniak (22prozentige wässrige Lösung) |
| 7,8 g | Ethanol |
| 0,3 g | Ascorbinsäure |
| 0,3 g | Ethylendiaminotetraessigsäure-Dinatriumsalz-Hydrat |
| ad 100,0 g | Wasser, vollentsalzt |

30 g der vorstehenden Färbelösung werden unmittelbar vor der Anwendung mit 30 g einer 6prozentigen wässrigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo ewaschen und getrocknet. Die Färbeergebnisse sind in Tabelle 5 zusammengefasst.

**Tabelle 2:**

| **Entwicklersubstanzen** | |
|---|---|
| **E8** | 1,4-Diaminobenzol |
| **E9** | 2,5-Diamino-phenylethanol-sulfat |
| **E10** | 3-Methyl-4-amino-phenol |
| **E11** | 4-Amino-2-aminomethyl-phenol-dihydrochlorid |
| **E13** | N,N-Bis(2'-hydroxyethyl)-p-phenylendiamin-sulfat |
| **E14** | 4,5-Diamino-1-(2'-hydroxyethyl)-pyrazol-sulfat |
| **E15** | 2,5-Diaminotoluol-sulfat |

**Tabelle 3:**

| **Direktziehende Farbstoffe** | |
|---|---|
| **D2** | 6-Chlor-2-ethylamino-4-nitro-phenol |
| **D3** | 2-Amino-6-chlor-4-nitro-phenol |

**Tabelle 4:**

| **Kupplersubstanzen** | |
|---|---|
| **K1** | 3-Amino-2-(1,3-benzodioxol-5-yl)phenol |
| **K2** | 3',6-Diamino-1,1'-biphenyl-2-ol |
| **K3** | 3-Amino-2-(3-pyridinyl)phenol |
| **K4** | 3-Amino-2-(4-pyridinyl)phenol |
| | |
| **K12** | 2-Amino-4-(2'-hydroxyethyl)amino-anisol-sulfat |
| **K13** | 1,3-Diamino-4-(2'-hydroxyethoxy)benzol-sulfat |
| **K14** | 2,4-Diamino-5-fluor-toluol-sulfat |
| **K18** | N-(3-Dimethylamino)phenylharnstoff |
| **K19** | 1,3-Bis(2,4-diaminophenoxy)propan-tetrahydrochlorid |
| **K21** | 3-Amino-phenol |
| **K22** | 5-Amino-2-methyl-phenol |
| **K23** | 3-Amino-2-chlor-6-methyl-phenol |
| **K24** | 5-Amino-4-fluor-2-methyl-phenol-sulfat |
| **K25** | 1-Naphthol |
| **K31** | 1,3-Dihydroxy-benzol |
| **K32** | 2-Methyl-1,3-dihydroxy-benzol |
| **K33** | 1-Chlor-2,4-dihydroxy-benzol |
| **K34** | 4-(2'-Hydroxyethyl)amino-1,2-methylendioxybenzolhydrochlorid |
| **K36** | 2-Amino-5-methyl-phenol |

**Tabelle 5: Haarfärbemittel**

| **Beispiel Nr.** | **35** | **36** | **37** | **38** | **39** | **40** |
|---|---|---|---|---|---|---|
| **Farbstoffe** | (Farbstoffmenge in Gramm) | | | | | |
| **K1** | 0,10 | 0,12 | 0,05 | 0,07 | 0,10 | 0,12 |
| **E8** | 0,30 | | | | | |
| **E9** | | | | | 0,25 | 0,20 |
| **E10** | | | | | | 0,10 |
| **E15** | | 0,25 | 0,30 | 0,25 | | |
| **K12** | | | 0,05 | | | |
| **K13** | | | | 0,05 | | |
| **K21** | 0,05 | | | | | |
| **K22** | | 0,05 | | | | |
| **K23** | | | 0,05 | 0,10 | 0,10 | 0,10 |
| **K25** | | | | | 0,10 | |
| **K31** | 0,20 | | | 0,15 | 0,10 | 0,10 |
| **K32** | | 0,20 | | 0,10 | | |
| **K33** | | | 0,20 | | | |
| **K36** | | | | | | 0,10 |
| **Färbeergebnis** | blond | blond | blond | blond | blond | blond |

| **Beispiel Nr.** | **41** | **42** | **43** | **44** | **45** | **46** |
|---|---|---|---|---|---|---|
| **Farbstoffe** | (Farbstoffmenge in Gramm) | | | | | |
| **K2** | 0,10 | 0,12 | 0,05 | 0,07 | 0,10 | 0,12 |
| **E8** | 0,30 | | | | | |
| **E9** | | | | | 0,25 | 0,20 |
| **E10** | | | | | | 0,10 |
| **E15** | | 0,25 | 0,30 | 0,25 | | |
| **K12** | | | 0,05 | | | |
| **K13** | | | | 0,05 | | |
| **K21** | 0,05 | | | | | |
| **K22** | | 0,05 | | | | |
| **K23** | | | 0,05 | 0,10 | 0,10 | 0,10 |
| **K25** | | | | | 0,10 | |
| **K31** | 0,20 | | | 0,15 | 0,10 | 0,10 |
| **K32** | | 0,20 | | 0,10 | | |
| **K33** | | | 0,20 | | | |
| **K36** | | | | | | 0,10 |
| **Färbeergebnis** | blond | blond | blond | blond | blond | blond |

| **Beispiel Nr.** | **47** | **48** | **49** | **50** | **51** | **52** |
|---|---|---|---|---|---|---|
| **Farbstoffe** | (Farbstoffmenge in Gramm) | | | | | |
| **K3** | 0,10 | 0,12 | 0,05 | 0,07 | 0,10 | 0,12 |
| **E8** | 0,30 | | | | | |
| **E9** | | | | | 0,25 | 0,20 |
| **E10** | | | | | | 0,10 |
| **E15** | | 0,25 | 0,30 | 0,25 | | |
| **K12** | | | 0,05 | | | |
| **K13** | | | | 0,05 | | |
| **K21** | 0,05 | | | | | |
| **K22** | | 0,05 | | | | |
| **K23** | | | 0,05 | 0,10 | 0,10 | 0,10 |
| **K25** | | | | | 0,10 | |
| **K31** | 0,20 | | | 0,15 | 0,10 | 0,10 |
| **K32** | | 0,20 | | 0,10 | | |
| **K33** | | | 0,20 | | | |
| **K36** | | | | | | 0,10 |
| **Färbeergebnis** | blond | blond | blond | blond | blond | blond |

| **Beispiel Nr.** | **53** | **54** | **55** | **56** | **57** | **58** |
|---|---|---|---|---|---|---|
| **Farbstoffe** | (Farbstoffmenge in Gramm) | | | | | |
| **K4** | 0,10 | 0,12 | 0,05 | 0,07 | 0,10 | 0,12 |
| **E8** | 0,30 | | | | | |
| **E9** | | | | | 0,25 | 0,20 |
| **E10** | | | | | | 0,10 |
| **E15** | | 0,25 | 0,30 | 0,25 | | |
| **K12** | | | 0,05 | | | |
| **K13** | | | | 0,05 | | |
| **K21** | 0,05 | | | | | |
| **K22** | | 0,05 | | | | |
| **K23** | | | 0,05 | 0,10 | 0,10 | 0,10 |
| **K25** | | | | | 0,10 | |
| **K31** | 0,20 | | | 0,15 | 0,10 | 0,10 |
| **K32** | | 0,20 | | 0,10 | | |
| **K33** | | | 0,20 | | | |
| **K36** | | | | | | 0,10 |
| **Färbeergebnis** | blond | blond | blond | blond | blond | blond |

### Beispiele 59 bis 82: Haarfärbemittel

Es werden Haarfärbelösungen der folgenden Zusammensetzung hergestellt:

| | |
|---|---|
| X g | 3-Aminophenol-Derivat der Formel (I) |
| | (Kupplersubstanz **K1** bis **K4** gemäss Tabelle 4) |
| U g | Entwicklersubstanz **E8** bis **E15** gemäß Tabelle 2 |
| Y g | Kupplersubstanz **K11** bis **K36** gemäß Tabelle 4 |
| Z g | direktziehende Farbstoffe **D2** und/oder **D3** gemäß Tabelle 3 |
| 10,0 g | Laurylethersulfat (28prozentige wässrige Lösung) |
| 9,0 g | Ammoniak (22prozentige wässrige Lösung) |
| 7,8 g | Ethanol |
| 0,3 g | Ascorbinsäure |
| 0,3 g | Ethylendiaminotetraessigsäure-Dinatriumsalz-Hydrat |
| ad 100,0 g | Wasser, vollentsalzt |

30 g der vorstehenden Färbecreme werden unmittelbar vor der Anwendung mit 30 g einer 6prozentigen wässrigen Wasserstoffperoxidlösung vermischt. Anschließend wird das Gemisch auf das Haar aufgetragen. Nach einer Einwirkzeit von 30 Minuten bei 40 °C wird das Haar mit Wasser gespült, mit einem handelsüblichen Shampoo gewaschen und getrocknet. Die Färbeergebnisse sind in Tabelle 6 zusammengefasst.

**Tabelle 6: Haarfärbemittel**

| **Beispiel Nr.** | **59** | **60** | **61** | **62** | **63** | **64** |
|---|---|---|---|---|---|---|
| **Farbstoffe** | (Farbstoffmenge in Gramm) | | | | | |
| **K1** | 0,60 | 1,30 | 1,15 | 0,15 | 0,15 | 10,15 |
| **E8** | 1,50 | | | | | |
| **E11** | 0,10 | | | | | |
| **E13** | | 1,60 | | | | 0,70 |
| **E14** | | | | 0,10 | 0,10 | |
| **E15** | | | 1,80 | 0,70 | 0,70 | |
| **K12** | 0,50 | | | | | |
| **K14** | 0,10 | | | | | |
| **K18** | 0,05 | | | | | |
| **K19** | 0,10 | | | | | |
| **K23** | | | 0,05 | 0,10 | 0,10 | 0,10 |
| **K24** | 0,15 | | | | | |
| **K31** | 0,90 | 1,10 | 1,10 | 0,40 | 0,40 | 0,40 |
| **K34** | 0,10 | | | | | |
| **D2** | | | | 0,10 | 0,10 | 0,10 |
| **D3** | | | | 0,05 | 0,05 | 0,05 |
| **Färbeergebnis** | schwarz | schwarz | schwarz | braun | braun | braun |

| **Beispiel Nr.** | **65** | **66** | **67** | **68** | **69** | **70** |
|---|---|---|---|---|---|---|
| **Farbstoffe** | (Farbstoffmenge in Gramm) | | | | | |
| **K2** | 0,60 | 1,30 | 1,15 | 0,15 | 0,15 | 0,15 |
| **E8** | 1,50 | | | | | |
| **E11** | 0,10 | | | | | |
| **E13** | | 1,60 | | | | 0,70 |
| **E14** | | | | 0,10 | 0,10 | |
| **E15** | | | 1,80 | 0,70 | 0,70 | |
| **K12** | 0,50 | | | | | |
| **K14** | 0,10 | | | | | |
| **K18** | 0,05 | | | | | |
| **K23** | | | 0,05 | 0,10 | 0,10 | 0,10 |
| **K24** | 0,15 | | | | | |
| **K31** | 0,90 | 1,10 | 1,10 | 0,40 | 0,40 | 0,40 |
| **K34** | 0,10 | | | | | |
| **D2** | | | | 0,10 | 0,10 | 0,10 |
| **D3** | | | | 0,05 | 0,05 | 0,05 |
| **Färbeergebnis** | schwarz | schwarz | schwarz | braun | braun | braun |

| **Beispiel Nr.** | **71** | **72** | **73** | **74** | **75** | **76** |
|---|---|---|---|---|---|---|
| **Farbstoffe** | (Farbstoffmenge in Gramm) | | | | | |
| **K3** | 0,60 | 1,30 | 1,15 | 0,15 | 0,15 | 0,15 |
| **E8** | 1,50 | | | | | |
| **E11** | 0,10 | | | | | |
| **E13** | | 1,60 | | | | 0,70 |
| **E14** | | | | 0,10 | 0,10 | |
| **E15** | | | 1,80 | 0,70 | 0,70 | |
| **K12** | 0,50 | | | | | |
| **K14** | 0,10 | | | | | |
| **K18** | 0,05 | | | | | |
| **K23** | | | 0,05 | 0,10 | 0,10 | 0,10 |
| **K24** | 0,15 | | | | | |
| **K31** | 0,90 | 1,10 | 1,10 | 0,40 | 0,40 | 0,40 |
| **K34** | 0,10 | | | | | |
| **D2** | | | | 0,10 | 0,10 | 0,10 |
| **D3** | | | | 0,05 | 0,05 | 0,05 |
| **Färbeergebnis** | schwarz | schwarz | schwarz | braun | braun | braun |

| **Beispiel Nr.** | **77** | **78** | **79** | **80** | **81** | **82** |
|---|---|---|---|---|---|---|
| **Farbstoffe** | (Farbstoffmenge in Gramm) | | | | | |
| **K4** | 0,60 | 1,30 | 1,15 | 0,15 | 0,15 | 0,15 |
| **E8** | 1,50 | | | | | |
| **E11** | 0,10 | | | | | |
| **E13** | | 1,60 | | | | 0,70 |
| **E14** | | | | 0,10 | 0,10 | |
| **E15** | | | 1,80 | 0,70 | 0,70 | |
| **K12** | 0,50 | | | | | |
| **K14** | 0,10 | | | | | |
| **K18** | 0,05 | | | | | |
| **K23** | | | 0,05 | 0,10 | 0,10 | 0,10 |
| **K24** | 0,15 | | | | | |
| **K31** | 0,90 | 1,10 | 1,10 | 0,40 | 0,40 | 0,40 |
| **K34** | 0,10 | | | | | |
| **D2** | | | | 0,10 | 0,10 | 0,10 |
| **D3** | | | | 0,05 | 0,05 | 0,05 |
| **Färbeergebnis** | schwarz | schwarz | schwarz | braun | braun | braun |

Alle in der vorliegenden Anmeldung enthaltenen Prozentangaben stellen, soweit nicht anders angegeben, Gewichtsprozente dar.

## Patentansprüche

1. 3-Aminophenol-Derivate der vorstehenden Formel (I), oder deren physiologisch verträglichen wasserlösliche Salze, 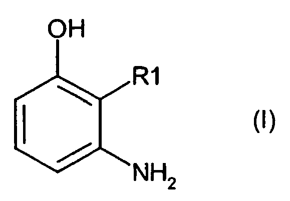 worin **R1** gleich einem Rest der Formel (II) oder (III) ist; 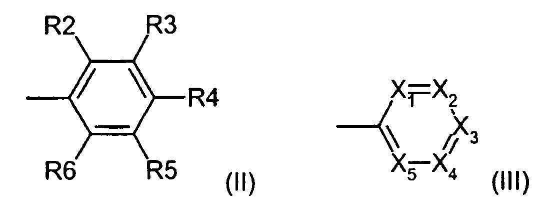 wobei **R2, R3, R4, R5** und **R6** unabhängig voneinander Wasserstoff, ein Halogenatom, eine Cyanogruppe, eine Hydroxygruppe, eine C₁-C₄-Alkoxygruppe, eine Phenoxygruppe, eine C₂-C₄-Hydroxyalkoxy-gruppe, eine C₁-C₆-Atkytgruppe, eine Phenylgruppe, eine C₁-C₄-Alkyl-thioethergruppe, eine Mercaptogruppe, eine Nitrogruppe, eine Amino-gruppe, eine C₁-C₄-Alkylaminogruppe, eine Hydroxy(C₂-C₄)alkylamino-gruppe, eine Di(C₁-C₄)alkylaminogruppe, eine Di(hydroxy(C₂-C₄)alkyl)-aminogruppe, eine (Dihydroxy(C₃-C₄)alkyl)-aminogruppe, eine (Hydroxy(C₂-C₄)alkyl)-C₁-C₄-alkylaminogruppe, eine Trifluoromethangruppe, eine Formylgruppe, eine Acetylgruppe, eine Trifluoroacetylgruppe, eine Trimethylsilylgruppe, eine (C₁-C₄)-Hydroxyalkylgruppe, eine (C₂-C₄)-Dihydroxyalkylgruppe, eine (C₁-C₄)-Aminoalkylgruppe, oder eine (C₁-C₄)-Cyanoalkylgruppe darstellen, oder zwei nebeneinanderliegende Reste R2 bis R6 jeweils zusammen mit dem Restmolekül einen heterozyklischen oder carbozyklischen, substituierten oder unsubstituierten Ring bilden;
**X₁**, **X₂, X₃, X₄** und **X₅** unabhängig voneinander gleich Stickstoff oder einer C-R7-Gruppe, C-R8-Gruppe, C-R9-Gruppe, C-R10-Gruppe oder C-R11-Gruppe sind, unter der Bedingung, dass mindestens einer und höchstens drei der Reste **X1** bis **X5** Stickstoff bedeuten; und
**R7, R8, R9, R10** und **R11** unabhängig voneinander Wasserstoff, ein Halogenatom, eine Cyanogruppe, eine C₁-C₆-Alkylgruppe, eine (C₁-C₄)-Alkylthioethergruppe, eine Mercaptogruppe, eine Nitrogruppe, eine Aminogruppe, eine (C₁-C₄)-Alkylaminogruppe, eine Hydroxy(C₂-C₄)alkylamino-gruppe, eine Di(C₁-C₄)alkylaminogruppe, eine Di(hydroxy(C₂₋C₄)-alkyl)-aminogruppe, eine (Dihydroxy(C₃₋C₄)alkyl)-aminogruppe, eine (Hydroxy(C₃-C₄)alkyl)-C₁-C₄-alkylaminogruppe, eine Trifluoromethangruppe, eine Formylgruppe, eine Acetylgruppe, eine Trifluoroacetylgruppe, eine Trimethylsilylgruppe, eine Carbamoylgruppe, eine (C₁-C₄)-Hydroxyalkylgruppe oder eine (C₂-C₄)-Dihydroxyalkylgruppe darstellen.

2. 3-Aminophenol-Derivat nach Anspruch 1, **dadurch gekennzeichnet, dass** es ausgewählt ist aus 6-Amino-[1,1'-biphenyl]-2-ol, 6-Amino-4'-methyl- [1,1'-biphenyl]-2-ol, 6-Amino-3'-methyl-[1,1'-biphenyl]-2-ol, 6-Amino-2'-methyl-[1,1'-biphenyl]-2-ol, 6-Amino-2',3'-dimethyl-[1,1'-biphenyl]-2-ol, 6-Amino-2',4'-dimethyl-[1,1'-biphenyl]-2-ol, 6-Amino-2',5'-dimethyl-[1,1'-biphenyl]-2-ol, 6-Amino-2',6'-dimethyl-[1,1'-biphenyl]-2-ol, 6-Amino-3',4'-dimethyl-[1,1'-biphenyl]-2-ol, 6-Amino-3',5'-dimethyl-[1,1'-biphenyl]-2-ol, 6-Amino-3',6'-dimethyl-[1,1'-biphenyl]-2-ol, 6-Amino-2',4',5'-trimethyl-[1,1'-biphenyl]-2-ol, 6-Amino-2',4',6'-trimethyl-[1,1'-biphenyl]-2-ol, 6-Amino-2',3',4'-trimethyl-[1,1'-biphenyl]-2-ol, 6-Amino-2',3',5'-trimethyl-[1,1'-biphenyl]-2-ol, 6-Amino-2',3',6'-trimethyl-[1,1'-biphenyl]-2-ol, 6-Amino-4'-chlor-[1,1'-biphenyl]-2-ol, 6-Amino-3'-chlor-[1,1'-biphenyl]-2-ol, 6-Amino-2'-chlor-[1,1'-biphenyl]-2-ol, 6-Amino-4'-fluor-[1,1'-biphenyl]-2-ol, 6-Amino-3'-fluor-[1,1'-biphenyl]-2-ol, 6-Amino-2'-fluor-[1,1'-biphenyl]-2-ol, 6-Amino-4'-brom-[1,1'-biphenyl]-2-ol, 6-Amino-3'-brom-[1,1'-biphenyl]-2-ol, 6-Amino-2'-brom-[1,1'-biphenyl]-2-ol, 6-Amino-3',5'-dichlor-[1,1'-biphenyl]-2-ol, 6-Amino-3',5'-difluor-[1,1'-biphenyl]-2-ol, 6-Amino-3'-brom-5'-methyl-1,1'-biphenyl-2-ol, 6-Amino-4'-(trifluoromethyl)-[1,1'-biphenyl]-2-ol, 6-Amino-3'-(trifluoromethyl)-[1,1'-biphenyl]-2-ol, 6-Amino-2'-(trifluoromethyl)-[1,1'-biphenyl]-2-ol, 6-Amino-4'-nitro-[1,1'-biphenyl]-2-ol, 6-Amino-3'-nitro-[1,1'-biphenyl]-2-ol, 6-Amino-2'-nitro-[1,1'-biphenyl]-2-ol, 6-Amino-5'-methyl-3'-nitro[1,1'-biphenyl]-2-ol, 6-Amino-4'-methyl-3'-nitro[1,1'-biphenyl]-2-ol, 6-Amino-2'-methyl-3'-nitro-[1,1'-biphenyl]-2-ol, 6-Amino-2'-nitro-4'-(trifluoromethyl)[1,1'-biphenyl]-2-ol, 6-Amino-3'-nitro-5'-(trifluoromethyl)[1,1'-biphenyl]-2-ol, 6-Amino-3'-nitro-4'-(trifluoromethyl)[1,1'-biphenyl]-2-ol, 6-Amino-3'-nitro-2'-(trifluoromethyl)-[1,1'-biphenyl]-2-ol, 6'-Amino-2'-hydroxy-[1,1'-biphenyl]-4-carbonitril, 6'-Amino-2'-hydroxy-[1,1'-biphenyl]-3- carbonitril, 6-Amino-4'-methoxy-[1,1'-biphenyl]-2-ol, 6-Amino-3'-methoxy-[1,1'-biphenyl]-2-ol, 6-Amino-2'-methoxy-[1,1'-biphenyl]-2-ol, 6-Amino-4'-ethoxy-[1,1'-biphenyl]-2-ol, 6-Amino-3'-ethoxy-[1,1'-biphenyl]-2-ol, 6-Amino-2'-ethoxy-[1,1'-biphenyl]-2-ol, 6-Amino-3',4'-dimethoxy-[1,1'-biphenyl]-2-ol, 6-Amino-3',5'-dimethoxy-[1,1'-biphenyl]-2-ol, 6-Amino-2',3'-dimethoxy-[1,1'-biphenyl]-2-ol, 6-Amino-2',4'-dimethoxy-[1,1'-biphenyl]-2-ol, 6-Amino-2',5'-dimethoxy-[1,1'-biphenyl]-2-ol, 3-Amino-2-(1,3-benzodioxol-5-yl)-phenol, 6-Amino-4'-methoxy-3'-methyl-[1,1'-biphenyl]-2-ol, 6-Amino-4'-methoxy-2'-nitro-[1,1'-biphenyl]-2-ol, 6-Amino-4'-methoxy-3'-nitro-[1,1'-biphenyl]-2-ol, 6-Amino-4'-phenoxy-[1,1'-biphenyl]-2-ol, 6-Amino-4'-methylthio-[1,1'-biphenyl]-2-ol, 6-Amino-3'-methylthio-[1,1'-biphenyl]-2-ol, 6-Amino-2'-methylthio-[1,1'-biphenyl]-2-ol, 6-Amino-[1,1'-biphenyl]-2,4'-diol, 6-Amino-[1,1'-biphenyl]-2,3'-diol, 6-Amino-[1,1'-biphenyl]-2,2'-diol, 2,2',3'-Trihydroxy-6-amino-[1,1'-biphenyl], 2,2',4'-Trihydroxy-6-amino-[1,1'-biphenyl], 2,2',5'-Trihydroxy-6-amino-[1,1'-biphenyl], 2,2',6'-Trihydroxy-6-amino-[1,1'-biphenyl], 2,3',4'-Trihydroxy-6-amino-[1,1'-biphenyl], 2,3',5'-Trihydroxy-6-amino-[1,1'-biphenyl], 6-Amino-2'-methyl-[1,1'-biphenyl]-2,4'-diol, 2',6-Diamino-[1,1'-biphenyl]-2-ol, 3',6-Diamino-[1,1'-biphenyl]-2-ol, 4',6-Diamino-[1,1'-biphenyl]-2-ol, 4',6-Diamino-[1,1'-biphenyl]-2,2'-diol, 3',6-Diamino-[1,1'-biphenyl]-2,2'-diol, 3',6-Diamino-[1,1'-biphenyl]-2,4'-diol, 3',6-Diamino-[1,1'-biphenyl]-2,5'-diol, 3',6-Diamino-[1,1'-biphenyl]-2,6'-diol, 2',3',6-Triamino-[1,1'-biphenyl]-2-ol, 2',4',6-Triamino-[1,1'-biphenyl]-2-ol, 2',5',6-Triamino-[1,1'-biphenyl]-2-ol, 2',6,6'-Triamino-[1,1'-biphenyl]-2-ol, 3',4',6-Triamino-[1,1'-biphenyl]-2-ol, 3',5',6-Triamino-[1,1'-biphenyl]-2-ol, 1-(6'-Amino-2'-hydroxy-1,1'-biphenyl-4-yl)ethanon, 6-Amino-1,1',3',1"-terphenyl-2-ol, 6-Amino-1,1':4',1"-terphenyl-2-ol, 6-Amino-4'-(aminomethyl)-1,1'-biphenyl-2-ol, 6-Amino-3'-(aminomethyl)-1,1'-biphenyl-2-ol, 6-Amino-2'-(aminomethyl)-1,1'-biphenyl-2-ol, (6'-Amino-2'-hydroxy-1,1'-biphenyl-4-yl)acetonitril; (6'-Amino-2'-hydroxy-1,1'-biphenyl-3-yl)acetonitril, (6'-Amino-2'-hydroxy-1,1'-biphenyl-2-yl)acetonitril, 6'-Amino-2'-hydroxy-1,1'-biphenyl-2-carbaldehyd, 6'-Amino-2'-hydroxy-1,1'-biphenyl-3-carbaldehyd, 6'-Amino-2'-hydroxy-1,1'-biphenyl-4-carbaldehyd, 3-Amino-2-(1-naphthyl)-phenol, 3-Amino-2-(2-naphthyl)phenol, 3-Amino-2-(2,3-dihydro-benzo[1,4]dioxin-6-yl)-phenol, 3-Amino-2-(2,3-dihydro-benzo[1,4]-dioxin-5-yl)-phenol, 3-Amino-2-(2,3-dihydro-benzofuran-5-yl)-phenol, 3-Amino-2-(2,3-dihydro-benzofuran-4-yl)-phenol, 3-Amino-2-(4-pyridinyl)-phenol, 3-Amino-2-(3-pyridinyl)-phenol, 3-Amino-2-(2-pyridinyl)-phenol, 3-Amino-2-(3-methyl-2-pyridinyl)-phenol, 3-Amino-2-(4-methyl-2-pyridinyl)-phenol, 3-Amino-2-(5-methyl-2-pyridinyl)-phenol, 3-Amino-2-(6-methyl-2-pyridinyl)-phenol, 3-Amino-2-(3-chlor-2-pyridinyl)-phenol, 3-Amino-2-(4-chlor-2-pyridinyl)-phenol, 3-Amino-2-(5-chlor-2-pyridinyl)-phenol, 3-Amino-2-(6-chlor-2-pyridinyl)-phenol, 3-Amino-2-(3-fluor-2-pyridinyl)-phenol, 3-Amino-2-(4-fluor-2-pyridinyl)-phenol, 3-Amino-2-(5-fluor-2-pyridinyl)-phenol, 3-Amino-2-(6-fluor-2-pyridinyl)-phenol, 3-Amino-2-(3-trifluoro-methyl-2-pyridinyl)-phenol, 3-Amino-2-(4-trifluoromethyl-2-pyridinyl)-phenol, 3-Amino-2-(5-trifluoromethyl-2-pyridinyl)-phenol, 3-Amino-2-(6-trifluoro-methyl-2-pyridinyl)-phenol, 3-Amino-2-(3-nitro-2-pyridinyl)-phenol, 3-Amino-2-(4-nitro-2-pyridinyl)-phenol, 3-Amino-2-(5-nitro-2-pyridinyl)-phenol, 3-Amino-2-(6-nitro-2-pyridinyl)-phenol, 3-Amino-2-(2-methyl-3-pyridinyl)-phenol, 3-Amino-2-(4-methyl-3-pyridinyl)-phenol, 3-Amino-2-(5-methyl-3-pyridinyl)-phenol, 3-Amino-2-(6-methyl-3-pyridinyl)-phenol, 3-Amino-2-(2-chlor-3-pyridinyl)-phenol, 3-Amino-2-(4-chlor-3-pyridinyl)-phenol, 3-Amino-2-(5-chlor-3-pyridinyl)-phenol, 3-Amino-2-(6-chlor-3-pyridinyl)-phenol, 3-Amino-2-(2-brom-3-pyridinyl)-phenol, 3-Amino-2-(4-brom-3-pyridinyl)-phenol, 3-Amino-2-(5-brom-3-pyridinyl)-phenol, 3-Amino-2-(6-brom-3-pyridinyl)-phenol, 3-Amino-2-(2-nitro-3-pyridinyl)-phenol, 3-Amino-2-(4-nitro-3-pyridinyl)-phenol, 3-Amino-2-(5-nitro-3-pyridinyl)-phenol, 3-Amino-2-(6-nitro-3-pyridinyl)-phenol, 3-Amino-2-(5-pyrimidinyl)-phenol und 3-Amino-2-(4-pyrimidinyl)-phenol sowie deren physiologisch verträglichen wasserlöslichen Salze.

3. 3-Aminophenol-Derivat nach Anspruch 1, **dadurch gekennzeichnet, dass** in der Formel (I) gilt: (i) **R1** ist gleich einem Rest der Formel (II) mit **R2** oder **R6** gleich Wasserstoff, oder (ii) **R1** ist gleich einem Rest der Formel (III) mit **X1** oder **X5** gleich **C-R7** beziehungsweise **C-R11,** wobei **R7** beziehungsweise **R11** gleich Wasserstoff sind.

4. 3-Aminophenol-Derivat nach einem der Ansprüche 1 bis 3, **dadurch gekennzeichnet, dass** es ausgewählt ist aus 6-Amino-3'-methoxy-[1,1'-biphenyl]-2-ol, 6-Amino-[1,1'-biphenyl]-2,4'-diol, 3-Amino-2-(2,3-dihydro-benzo[1,4]dioxin-6-yl)-phenol, 3-Amino-2-(2,3-dihydro-benzofuran-5-yl)-phenol, 3-Amino-2-(3-pyridinyl)-phenol, 3-Amino-2-(4-pyridinyl)-phenol und 3-Amino-2-(5-pyrimidinyl)-phenol sowie deren physiologisch verträglichen wasserlöslichen Salze.

5. Mittel zur Färbung von Keratinfasern auf der Basis einer Entwicklersubstanz-Kupplersubstanz-Kombination, **dadurch gekennzeichnet, dass** es als Kupplersubstanz mindestens ein 3-Aminophenol-Derivat der Formel (I) nach einem der Ansprüche 1 bis 4 enthält.

6. Mittel nach Anspruch 5, **dadurch gekennzeichnet, dass** das 3-Aminophenol-Derivat der Formel (I) in einer Menge von 0,005 bis 20 Gewichtsprozent enthalten ist.

7. Mittel nach Anspruch 5 oder 6, **dadurch gekennzeichnet, dass** die Entwicklersubstanz ausgewählt ist aus der Gruppe bestehend aus 1,4-Diamino-benzol, 1,4-Diamino-2-methyl-benzol, 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(2-thienyl)benzol, 1,4-Diamino-2-(3-thienyl)benzol, 1,4-Diamino-2-(pyridin-3-yl)benzol, 2,5-Diamino-biphenyl, 1,4-Diamino-2-methoxymethyl-benzol, 1,4-Diamino-2-amino-methylbenzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)amino]-anilin, 4-[Di(2-hydroxyethyl)amino]-anilin, 4-[Di(2-hydroxyethyl)amino]-2-methylanilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(1-hydroxyethyl)-benzol, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluor-phenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methylphenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1H-pyrazol, 4,5-Diamino-1-[(4-methylphenyl)methyl]-1H-pyrazol, 1-[(4-Chlorphenyl)methyl]-4,5-diamino-1H-pyrazol, 4,5-Diamino-1-methyl-1H-pyrazol, 2-Amino-phenol, 2-Amino-6-methyl-phenol, 2-Amino-5-methyl-phenol und 1,2,4-Trihydroxy-benzol.

8. Mittel nach einem der Ansprüche 5 bis 7, **dadurch gekennzeichnet, dass** es zusätzlich zu den Verbindungen der Formel (I) mindestens eine weitere bekannte Kupplersubstanzen enthält, welche ausgewählt ist aus der Gruppe bestehend aus N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxy-ethoxy)-benzol, 1,3-Diamino-4-(2,3-dihydroxypropoxy)-benzol, 1,3-Diamino-4-(3-hydroxypropoxy)-benzol, 1,3-Diamino-4-(2-methoxy-ethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)amino]-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion.

9. Mittel nach einem der Ansprüche 5 bis 8, **dadurch gekennzeichnet, dass** die Entwicklersubstanzen und Kupplersubstanzen, bezogen auf die Gesamtmenge des Färbemittels, jeweils in einer Gesamtmenge von 0,005 bis 20 Gewichtsprozent enthalten sind.

10. Mittel nach einem der Ansprüche 5 bis 9 , **dadurch gekennzeichnet, dass** es zusätzlich mindestens einen direktziehenden Farbstoff enthält.

11. Mittel nach einem der Ansprüche 5 bis 10, **dadurch gekennzeichnet, dass** es einen pH-Wert von 6,5 bis 11,5 aufweist.

12. Gebrauchsfertiges Mittel zur oxidativen Färbung von Keratinfasern, welches in einem zum Färben geeigneten Medium mindestens eine Entwicklersubstanz und mindestens eine Kupplersubstanz sowie mindestens ein Oxidationsmittel enthält, **dadurch gekennzeichnet, dass** es als Kupplersubstanz mindestens ein 3-Aminophenol-Derivat der Formel (I) nach einem der Ansprüche 1 bis 4 enthält.

13. Mittel nach einem der Ansprüche 5 bis 12, **dadurch gekennzeichnet, dass** es ein Haarfärbemittel ist.

14. Verfahren zum oxidativen Färben von Haaren, insbesondere menschlichen Haaren, **dadurch gekennzeichnet, dass** man vor der Anwendung ein Haarfärbemittel nach einem der Ansprüche 5 bis 13 mit einem Oxidationsmittel vermischt, sodann auf das Haar aufträgt, bei einer Temperatur von 15 bis 50 °C 10 bis 45 Minuten lang einwirken lässt, das Haar anschliessend mit Wasser ausspült, gegebenenfalls shampooniert und sodann trocknet.

## Claims

1. 3-amino phenol derivatives of the above formula (1), or their physiologically compatible water soluble salts, 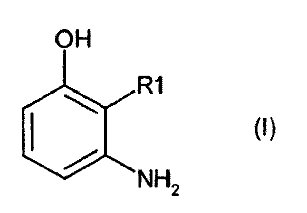 wherein **R1** equals a group of the formula (II) or (III); 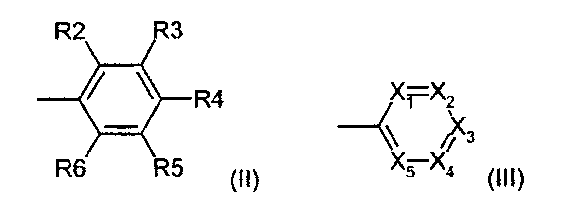 wherein **R2, R3, R4, R5** and **R6** represent independently of one another hydrogen, a halogen atom, a cyano group, a hydroxy group, a C₁-C₄ alkoxy group, a phenoxy group, a C₂-C₄ hydroxy alkoxy group, a C₁-C₆ alkyl group, a phenyl group, a C₁-C₄ alkyl thioether group, a C₁-C₄ alkyl amino group, a hydroxy (C₂-C₄) alkyl amino group, a di(C₁-C₄) alkyl amino group, a di(hydroxy (C₂-C₄) alkyl) amino group, a Di(hydroxy (C₃-C₄) alkyl) amino group, a (hydroxy (C₂-C₄) alkyl) C₁-C₄ alkyl amino group, a trifluoro methane group, a formyl group, an acetyl group, a trifluoro acetyl group, a trimethyl silyl group, a (C₁-C₄) hydroxy alkyl group, a (C₂-C₄) alkyl) C₁-C₄ dihydroxy alkyl group, a (C₁-C₄) amino alkyl amino group, or a (C₁-C₄) alkyl) cyano alkyl group, or two groups R2 through R6 that lie next to one another, in each instance forming, together with the group molecule, a heterocyclic or carbocyclic, substituted or unsubstituted ring;
**X₁, X₂, X₃, X₄** and **X₅** independently of one another equal nitrogen or a C-R7 group, C-R8 group, C-R9 group, C-R10 group or C-R11 group, under the condition that at least one and no more than three of the groups **X₁** through **X₅** signify nitrogen; and
**R7, R8, R9, R10** and **R11** represent independently of one another [equal] hydrogen, a halogen atom, a cyano group, a C₁-C₆ alkyl thioether group, a mercapto group, a nitro group, an amino group, a (C₁-C₄) alkyl amino group, a hydroxy (C₂-C₄) alkyl amino group, a di(C₁-C₄) alkyl amino group, a di(hydroxy (C₂-C₄) alkyl) amino group, a (dihydroxy (C₃-C₄) alkyl) amino group, a (hydroxy (C₂-C₄) alkyl) C₁-C₄ alkyl amino group, a trifluoro methane group, a formyl group, an acetyl group, a trifluoro acetyl group, a trimethyl silyl group, a carbamoyl group, a (C₁-C₄) hydroxy alkyl group or a (C₂-C₄) dihydroxy alkyl group.

2. 3-amino phenol derivative according to Claim 1, **characterized in that** it is chosen from [the group consisting of] 6-amino [1,1'-biphenyl]-2-ol, 6-amino-4' methyl [1,1'-biphenyl]-2-ol, 6-amino 3' methyl [1,1'-biphenyl]-2-ol, 6-amino-2' methyl [1,1'-biphenyl]-2-ol, 6-amino-2',3' dimethyl [1,1'-biphenyl]-2-ol, 6-amino-2',4' dimethyl [1,1'-biphenyl]-2-ol, 6-amino-2',5' dimethyl [1,1'-biphenyl]-2-ol, 6-amino-2',6' dimethyl [1,1'-biphenyl]-2-ol, 6-amino-3',4' dimethyl [1,1'-biphenyl]-2-ol, 6-amino-3',5' dimethyl [1,1'-biphenyl]-2-ol, 6-amino-3',6' dimethyl [1,1'-biphenyl]-2-ol, 6-amino-2',45' trimethyl [1,1'-biphenyl]-2-ol, 6-amino-2',3',5' trimethyl [1,1'-biphenyl]-2-ol, 6-amino-2',3',6' trimethyl [1,1'-biphenyl]-2-ol, 6-amino-4' chlor[1,1'-biphenyl]-2-ol, 6-amino-3' chlor[1,1'-biphenyl]-2-ol, 6-amino-2' chlor[1,1'-biphenyl]-2-ol, 6-amino-4' fluor[1,1'-biphenyl]-2-ol, 6-amino-3' fluor[1,1'-biphenyl]-2-ol, 6-amino-2' fluor[1,1'-biphenyl]-2-ol, 6-amino-4' brom[1,1'-biphenyl]-2-ol, 6-amino-3' brom[1,1'-biphenyl]-2-ol, 6-amino-2' brom[1,1'-biphenyl]-2-ol, 6-amino-3',5' dichlor[1,1'-biphenyl]-2-ol, 6-amino-3'5' difluor[1,1'-biphenyl]-2-ol, 6-amino-3'-brom-5' methyl 1,1' biphenyl-2-ol, 6-amino-4'-(trifluoro methyl)-[1,1'-biphenyl]-2-ol, 6-amino-3'-(trifluoro methyl)-[1,1'-biphenyl]-2-ol, 6-ammo-2'-(trifluoro methyl)-[1,1'-biphenyl]-2-ol, 6-amino-4'-nitro-[1,1'-biphenyl]-2-ol, 6-ammo-3'-nitro-[1,1'-biphenyl]-2-ol, 6-amino-2'-mitro-[1,1'-biphenyl]-2-ol, 6-amiono-3'-nitro-[1.1'-biphenyl]-2-ol, 6-amino-4'-methyl-3'-nitro[1.1'-biphenyl]-2-ol, 6-amino-2'-methyl-3'-nitro-[1,1'-biphenyl]-2-ol, 6-amino-2'-nitro-4'-(trifluoro methyl)[1,1'-biphenyl]-2-ol, 6-amino-3'-nitro-5'-(trifluoro methyl)[1,1'-biphenyl]-2-ol, 6-amino-3'-nitro-4'-(trifluoro methyl)[1,1'-biphenyl]-2-ol, 6-amino-3'-nitro-2'-(trifluoro methyl)-[1,1'-biphenyl]-2-ol, 6-amino-2'-hydroxy-[1,1'-biphenyl]-4-carbon nitrile, 6-amino-2'-hydroxy-[1,1'-biphenyl]-3-carbon nitrile, 6-amino-4'-methoxy-[1,1'-biphenyl]-2-ol, 6-amino-34'-methoxy-[1,1'-biphenyl]-2-ol, 6-amino-2'-methoxy-[1,1'-biphenyl]-2-ol, 6-amino-4'-ethoxy-[1,1'-biphenyl]-2-o1, 6-amino-3'-ethoxy-[1,1'-biphenyl]-2-ol, 6-amino-2'-ethoxy-[1,1'-biphenyl]-2-ol, 6-amino-3',4'-dimethoxy-[1,1'-biphenyl]-2-ol, 2',4'-dimethoxy-[1,1'-biphenyl]-2-ol, 6-amino-23',5'-dimethoxy-[1,1'-biphenyl]-2-ol, 36-amino-2-(1,3-benzodioxol-5-yl)-phenol, 6-amino-4'-methoxy-3'-methyl-[1,1'-biphenyl]-2-ol, 6-amino-4'-methoxy-2'-nitro-[1,1'-biphenyl]-2-ol, 6-ammo-4'-methoxy-32'-nitro-[1,1'-biphenyl]-2-ol, 6-amino-4'-phenoxy-[1,1'-biphenyl]-2-ol, 6-amino-4'-methyl thio-[1,1'-biphenyl]-2-ol, 6-amino-3'-methyl thio-[1,1'-biphenyl]-2-ol, 6-amino-2'-methyl thio-[1,1'-biphenyl]-2-ol, 6-amino-[1,1'-biphenyl]-2,4'-diol, 6-amino[1,1'-biphenyl]2,3'-diol, 6-amino[1,1'-biphenyl]2,2'-diol, 2,2',3'-trihydroxy-6-amino [1,1'-biphenyl], 2,2',4'-trihydroxy-6-amino [1,1'-biphenyl], 2,2',5'-trihydroxy-6-amino [1,1'-biphenyl], 2,2',6'-trihydroxy-6-amino [1,1'-biphenyl, 2,3'4'-trihydroxy-6-amino [1,1'-biphenyl], 2,3',5'-trihydroxy-6-amino-[1,1'-biphenyl], 6-amino-2'-methyl [1,1'-biphenyl]-2,4'-diol, 2',6-diamino [1,1'-biphenyl]-2-ol, 3'6-diamino [1,1'-biphenyl]-2-ol, 4'6-diamino [1,1'-biphenyl]-2-ol, 4',6-diamino [1,1'-biphenyl]-2,2'-diol, 3',6-diamino [1,1'-biphenyl]-2,2'-diol, 3',6-diamino [1,1'-biphenyl]-2,4'-diol, 3'6-diamino [1,1'-biphenyl]-2,5'-diol, 3'6-diamino[1,1'-biphenyl]-2,6'-diol, 2',3'6-triamino [1,1'-biphenyl]-2-ol, 2',4'6-triamino [1,1'-biphenyl]-2-ol, 2',5'6-triamino [1,1'-biphenyl]-2-ol, 2',6'6-triamino [1,1'-biphenyl]-2-ol, 3',4',6-triamino [1,1'-biphenyl]-2-ol, 3',45',6-triamino [1,1'-biphenyl]-2-ol, 1-(6'-amino-2'hydroxy-1,1'-biphenyl-4-yl)ethanone, 6-amino-1,1',3',1"-terphenyl-2-ol, 6-amino-1,1':4',1"-terphenyl-2-ol, 6-amino-4'-(amino methyl)-1,1'-biphenyl-2-ol, 6-amino-3'-(amino methyl)-1,1'-biphenyl-2-ol, 6-amino-2'-(amino methyl)-1,1'-biphenyl-2-ol, (6'-amino-2'-hydroxy-1,1'-biphenyl-4-yl) acetone nitrile; (6'-amino-2'-hydroxy-1,1'-biphenyl-3-yl) acetone nitrile, (6'-amino-2'-hydroxy-1,1'-biphenyl-2-yl) acetone nitrile, (6'-amino-2'-hydroxy-1,1'-biphenyl-2-carbaldehyde, 6'-amino-2'-hydroxy-1,1'-biphenyl-3-carbaldehyde, 6'-amino-2'-hydroxy-1,1'-biphenyl-4-carbaldehyde, 3-amino-2-(1-naphthyl)-phenol, 3-amino-2-(2-naphthyl)-phenol, 3-amino-2-(2,3-dihydrobenzo[1,4]dioxin-6-yl)-phenol, 3-amino-2-(2,3-dihydro-benzo[1,4]dioxin-5-yl)-phenol, 3-amino-2-(2,3-dihydro-benzofuran-5-yl)-phenol, 3-amino-2-(2,3-dihydro-benzofuran-4-yl)-phenol, 3-amino-2-(4-pyridinyl)-phenol, 3-amino-2-(3-pyridinyl)-phenol, 3-amino-2-(2-pyridinyl)-phenol, 3-amino-2-(3-methyl-2-pyridinyl)-phenol, 3-amino-2-(4-methyl-2-pyridinyl)-phenol, 3-amino-2-(5-methyl-2-pyridinyl)-phenol, 3-amino-2-(6-methyl-2-pyridinyl)-phenol, 3-amino-2-(3-chlor-2-pyridinyl)-phenol, 3-amino-2-(4-chlor-2-pyridinyl)-phenol, 3-amino-2-(5-chlor-2-pyridinyl)-phenol, 3-amino-2-(6-chlor-2-pyridinyl)-phenol, 3-amino-2-(3-fluor-2-pyridinyl)-phenol, 3-amino-2-(4-fluor-2-pyridinyl)-phenol, 3-amino-2-(4-fluor-5-pyridinyl)-phenol, 3-amino-2-(6-fluor-2-pyridinyl)-phenol, 3-amino-2-(3-trifluoro-methyl-2-pyridinyl)-phenol, 3-amino-2-(4-trifluoro-methyl-2-pyridinyl)-phenol, 3-amino-2-(5-trifluoro-methyl-2-pyridinyl)-phenol, 3-amino-2-(6-trifluoro-methyl-2-pyridinyl)-phenol, 3-amino-2-(3-nitro-2-pyridinyl)-phenol, 3-amino-2-(4-nitro-2-pyridinyl)-phenol, 3-amino-2-(5-nitro-2-pyridinyl)-phenol, 3-amino-2-(6-nitro-2-pyridinyl)-phenol, 3-amino-2-(2-methyl-3-pyridinyl)-phenol, 3-amino-2-(42-methyl-3-pyridinyl)-phenol, 3-amino-2-(5-methyl-3-pyridinyl)-phenol, 3-amino-2-(6-methyl-3-pyridinyl)-phenol, 3-amino-2-(2-chlor-3-pyridinyl)-phenol, 3-amino-2-(4-chlor-3-pyridinyl)-phenol, 3-amino-2-(5-chlor-3-pyridinyl)-phenol, 3-amino-2-(64-chlor-3-pyridinyl)-phenol, 3-amino-2-(2-brom-3-pyridinyl)-phenol, 3-amino-2-(4-brom-3-pyridinyl)-phenol, 3-amino-2-(5-brom-3-pyridinyl)-phenol, 3-amino-2-(6-brom-3-pyridinyl)-phenol, 3-amino-2-(2-nitro-3-pyridinyl)-phenol, 3-amino-2-(4-nitro-3-pyridinyl)-phenol, 3-amino-2-(5-nitro-3-pyridinyl)-phenol, 3-amino-2-(6-nitro-3-pyridinyl)-phenol, 3-amino-2-(5-pyrimidinyl)-phenol and 3-amino-2-(4-pyrimidinyl)-phenol as well as their physiologically compatible water soluble salts.

3. 3-amino phenol derivative according to Claim 1, **characterized in that** in the formula (I): (i) **R1** equals a group of the formula (II) with **R2** or **R6** equal to hydrogen, or (ii) **R1** equals a group of the formula (III) with **X1** or X5 equal to **C-R7** or **C-R11,** respectively, wherein **R7** or **R11,** respectively, equal hydrogen.

4. 3-amino phenol derivative according to one of Claims 1 through 3, **characterized in that** it is chosen from [the group consisting of] 6-amino-3'-methoxy-[1,1'-biphenyl]2-ol, 6-amino-[1,1'-biphenyl]2,4'-diol, 3-amino-2-(2,3-dihydro-benzo[1,4]dioxin-6-yl)-phenol, 3-amino-2-(2,3-dihydro-benzofuran-5-yl)-phenol, 3-amino-2-(3-pyridinyl)-phenol, 3-amino-2-(4-pyridinyl)-phenol and 3-amino-2-(5-pyrimidinyl)-phenol as well as their physiologically compatible water soluble salts.

5. Agent for coloring keratin fibers on the basis of a developer substance/coupler substance combination, **characterized in that** as a coupler substance it contains at least one 3-amino phenol derivative of the formula (I) according to one of Claims 1 through 4.

6. Agent according to Claim 5, **characterized in that** the 3-amino phenol derivative of the formula (I) is contained in an amount from 0.005 to 20 percent by weight.

7. Agent according to Claim 5 or 6, **characterized in that** the developer substance is chosen from the group consisting of 1,4-diamino-benzene, 1,4-diamino-2-methyl benzene, 1,4-diamino-2,6-dimethyl benzene, 1,4-diamino-3,5-diethyl benzene, 1,4-diamino-2,5-dimethyl benzene, 1,4-diamino-2,3-dimethyl benzene, 2-chlor-1,4-diamino benzene, 1,4-(diamino-2-(2-thienyl) benzene, 1,4-(diamino-2-(3-thienyl) benzene, 1,4-diamino-2-(pyridine-3-yl) benzene, 2,5-diamino-biphenyl, 1,4-diamino-2-methoxy methyl benzene, 1,4-diamino-2-(2-hydroxy ethoxy) benzene, 2-(2-(acetyl amino) ethoxy)-1,4-diamino benzene, 4-phenyl amino aniline, 4-dimethyl amino aniline, 4-diethyl amino aniline, 4-dipropyl amino aniline, 4-[ethyl (2-hydroxy ethyl) amino] aniline, 4-[ethyl (2-hydroxy ethyl) amino] aniline, 4-[ethyl di(2-hydroxy ethyl) amino] aniline, 4-[di(2-hydroxy ethyl) amino]-2-methyl aniline, 4-[(2-methoxy ethyl) amino] aniline, 4-[(2-methoxy ethyl) amino] aniline, 4-[3-hydroxy propyl) amino] aniline, 4-[2,3-dihydroxy propyl) amino] aniline, 1,4-diamino-2-(1-hydroxy ethyl) benzene, 1,4-diamino-2-(21-hydroxy ethyl) benzene, 1,4-diamino-2-(1-methyl ethyl) benzene, 1,3-bis[(4-amino phenyl)(2-hydroxy ethyl)amino]-2-propanol, 1,4-bis[(4-amino phenyl)amino]-butane, 1,8-bis(2,5-diamino phenoxy)-3,6-dioxaoctane, 4-amino phenol, 4-amino-3-methyl phenol, 4-amino-3-(hydroxy methyl) phenol, 4-amino-3-fluor-phenol, 4-methyl amino phenol, 4-amino-2-(amino methyl) phenol, 4-amino-2-(hydroxy methyl) phenol, 4-amino-2-fluor phenol, 4-amino-2[(2-hydroxy ethyl) amino] methyl phenol, 4-amino-2-methyl phenol, 4-amino-2-(methoxy methyl) phenol, 4-amino-2-(2-hydroxy ethyl) phenol, 5-amino salicylic acid, 2,5-diamino pyridine, 2,4,5,6-tetra amino pyrimidine, 2,5,6-triamino-4-(1H) pyrimidone, 4,5-diamino-methyl phenyl) methyl]-1H pyrazole, 1-[(4-chlor phenyl) methyl]-4,5-diamino-1H-pyrazole, 4,5-diamino-1-methyl-1H-pyrazole, 2-amino phenol, 2-amino-6-methyl phenol, 2-amino-5-methyl phenol and 1,2,4-trihydroxy benzene.

8. Agent according to one of Claims 5 through 7, **characterized in that** it contains, in addition to the compounds of formula (I) at least one other known coupler substance that is chosen from the group consisting of N-(3-dimethyl amino phenyl) carbamide, 2,6-diamino pyridine, 2-amino-4-[2-hydroxy ethyl) amino] anisole, 2,4-diamino-1-fluor-5-methyl benzene, 2,4-diamino-1-methoxy-5-methyl benzene, 2,4-diamino-1-ethoxy-5-methyl benzene, 2,4-diamino-1-(2-hydroxy ethoxy)-5-methyl benzene, 2,4-di[(2-hydroxy ethyl) amino]-1,5-dimethoxy-benzene, 2,3-diamino-6-methoxy pyridine, 3-amino-6-methoxy-2-(methyl amino) pyridine, 2,6-diamino-3,5-dimethoxy pyridine, 3,5-diamino-2,6-dimethoxy pyridine, 1,3-diamino benzene, 2,4-diamino-1-(2-hydroxy ethoxy) benzene, 1,3-diamino-4-(2,3-dihydroxy propoxy) benzene, 1,3-diamino-4-(3-hydroxy propoxy) benzene, 1,3-diamino-4-(2-methoxy ethoxy) benzene, 2,4-diamino-1,5-di(2-hydroxy ethoxy) benzene, 1-(2-amino ethoxy)-2,4-diamino benzene, 2-amino-1-(2-hydroxy ethoxy)-4-methyl amino benzene, 2,4-diamino phenoxy acetic acid, 3-[see original for diagram](2-hydroxy ethyl) amino] aniline, 4-amino-2-di[2-hydroxy ethyl) amino]-1 ethoxy benzene, 5-methyl-2-(1-methyl ethyl) phenol, 3-[(2-hydroxy ethyl) amino] aniline, 3-[(2-amino ethyl) amino] aniline, 1,3-di(2,4-diamino phenoxy) propane, di(2,4-diamino phenoxy) methane, 1,3-diamino-2,4-dimethoxy benzene, 2,6-bis(2-hydroxy ethyl) amino toluol, 4-hydroxy indole, 3-dimethyl amino phenol, 3-diethyl amino phenol, 5-amino-2-methyl phenol, 5-amino-4-fluor-2-methyl phenol, 5-amino-4-methoxy-2-methyl phenol, 5-amino-4-ethoxy-2-methyl phenol, 3-amino-2,4-dichlor phenol, 5-amino-2,4-dichlor phenol, 3-amino phenol, 2-[(3-hydroxy phenyl) amino] acetamide, 5-[(2-hydroxy amino]-2-methyl phenol, 5-amino-2-ethyl phenol, 5-amino-2-methoxy phenol, 2-(4-amino-2-hydroxy phenoxy) ethanol, 5-[(3-hydroxy propyl) amino]-2-methyl pheneol, 3-[(2,3-dihydroxy propyl) amino]-2-methyl phenol, 3-[(2-hydroxy ethyl) amino]-2-methyl phenol, 2-amino-3-hydroxy pyridine, 2,6-dihydroxy-3,4-dimethyl pyridine, 5-amino-4-chlor-2-methyl phenol, 1-naphthol, 2-methyl-1-naphthol, 1,5-dihydroxy naphthaline, 1,7-dihydroxy naphthaline, 2,3-dihydroxy naphthaline, 2,7-dihydroxy naphthaline, 2-methyl-1-naphthol acetate, 1,3-dihydroxy benzene, 1-color-2,4-dihydroxy benzene, 2-chlor-1,3-dihydroxy benzene, 1,2-dichlor-3,5-dihydroxy-4-methyl benzene, 1,5-dichlor-2,4-dihydroxy benzene, 1,3-dihydroxy-2-methyl benzene, 3,4-methylene dioxy phenol, 3,4-methylene dioxy aniline, 5-[(2-hydroxy ethyl) amino]-1,3-benzodioxole, 6-brom-1-hydroxy-3,4-methylene dioxy benzene, 3,4-diamino benzoic acid, 3,4-dihydro-6-hydroxy-1,4(2H)-benzoxacine, 6-amino-3,4-dihydro-1,4(2H)-benzoxacine, 3-methyl-1-phenyl-5-pyrazolone, 5,6-dihydroxy indole, 5,6-dihydroxy indoline, 5-hydroxy indole, 6-hydroxy indole, 7-hydroxy indole and 2,3 indolindione.

9. Agent according to one of Claims 5 through 8, **characterized in that** the developer substances and coupler substances, based on the overall amount of the colorant, are in each case contained in an overall amount from 0.005 to 20 percent by weight.

10. Agent according to one of Claims 5 through 9, **characterized in that** it also contains at least one direct-drawing dye.

11. Agent according to one of Claims 5 through 10, **characterized in that** it has a pH value from 6.5 to 11.5.

12. Ready-made agent for the oxidative coloring of keratin fibers, which in a medium suitable for coloring contains at least one developer substance and at least one coupler substance as well as at least one oxidation agent, **characterized in that** it contains as a coupler substance at least one 3-amino phenol derivative of the formula (I) according to one of Claims 1 through 4.

13. Agent according to one of Claims 5 through 12, **characterized in that** it is a hair-coloring agent.

14. Method for the oxidative coloring of hair, in particular human hair, **characterized in that** prior to application, a hair-coloring agent according to one of Claims 5 through 13 is mixed with an oxidation agent and then applied on the hair and let sit to work for 45 minutes at a temperature from 15 to 50° C; the hair is then rinsed with water and, optionally, shampooed, and then dried.

## Revendications

1. Dérivés de 3-amino phénol de formule ci-dessus (1), ou leurs sels hydrosolubles physiologiquement compatibles, 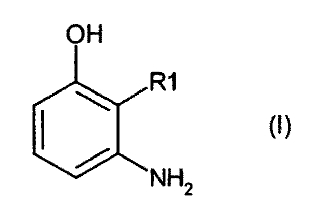 dans laquelle **R1** est égal à un groupe de formule (II) ou (III) ; 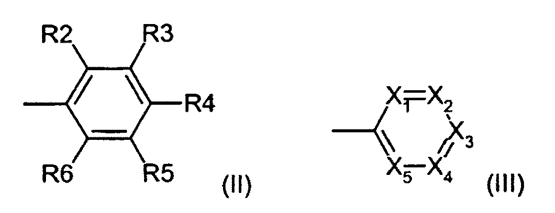 dans laquelle **R2, R3, R4, R5** et **R6** représentent indépendamment l'un de l'autre un hydrogène, un atome d'halogène, un groupe cyano, un groupe hydroxy, un groupe alcoxy en C1 à C4, un groupe phénoxy, un groupe hydroxy alcoxy en C2 à C4, un groupe alkyle en C1 à C6, un groupe phényle, un groupe alkyl en C1 à C4 thio-éther, un groupe alkyl en C1 à C4 amino, un groupe hydroxy alkyl en (C2 à C4) amino, un groupe di-alkyl en (C1 à C4) amino, un groupe di(hydroxy alkyl en (C2 à C4)) amino, un groupe di(hydroxy alkyl en (C3 à C4)) amino, un groupe (hydroxy alkyl en (C2 à C4)) alkyle en C1 à C4 amino, un groupe trifluoro méthane, un groupe formyle, un groupe acétyle, un groupe trifluoro acétyle, un groupe triméthyl silyle, un groupe hydroxy alkyle en (C1 à C4), un groupe (alkyl en (C2 à C4)) dihydroxy alkyle en C1 à C4, un groupe amino alkyle en (C1 à C4) amino, ou un groupe (alkyle en (C1 à C4)) cyano alkyle, ou deux groupes R2 à R6 qui se trouvent l'un près de l'autre, en formant dans chaque cas, conjointement avec la molécule du groupe, un cycle hétérocyclique ou carbocyclique, substitué ou non substitué ;
**X₁, X₂, X₃, X₄** et **X₅** indépendamment l'un de l'autre sont égal à un azote ou un groupe C-R7, un groupe C-R8, un groupe C-R9, un groupe C-R10 ou un groupe C-R11, à la condition qu'au moins un et pas plus de trois des groupes **X₁** à **X₅** signifient un azote ; et
**R7, R8, R9, R10** et **R11** représentent indépendamment l'un de l'autre [sont égaux à] un hydrogène, un atome d'halogène, un groupe cyano, un groupe alkyl en C₁ à C₆ thio-éther, un groupe mercapto, un groupe nitro, un groupe amino, un groupe alkyl en (C₁ à C₄) amino, un groupe hydroxy alkyl en (C₂ à C₄) amino , un groupe di-alkyl en (C₁ à C₄) amino, un groupe di(hydroxy alkyl en (C₂ à C₄)) amino, un groupe (dihydroxy alkyl en (C₃ à C₄)) amino, un groupe (hydroxy alkyl en (C₂ à C₄)) alkyl en C₁ à C₄ amino, un groupe trifluoro méthane, un groupe formyle, un groupe acétyle, un groupe trifluoro acétyle, un groupe triméthyl silyle, un groupe carbamoyle, un groupe hydroxy alkyle en (C₁ à C₄) ou un groupe dihydroxy alkyle en (C₂ à C₄).

2. Dérivé de 3-amino phénol selon la revendication 1, **caractérisé en ce qu'**il est choisi parmi [le groupe constitué de] 6-amino [1,1'-biphényl]-2-ol, 6-amino-4' méthyl [1,1'-biphényl]-2-ol, 6-amino 3' méthyl [1,1'-biphényl]-2-ol, 6-amino-2' méthyl [1,1'-biphényl]-2-ol, 6-amino-2',3' diméthyl [1,1'-biphényl]-2-ol, 6-amino-2',4' diméthyl [1,1'-biphényl]-2-ol, 6-amino-2',5' diméthyl [1,1'-biphényl]-2-ol, 6-amino-2',6' diméthyl [1,1'-biphényl]-2-ol, 6-amino-3',4' diméthyl [1,1'-biphényl]-2-ol, 6-amino-3',5' diméthyl [1,1'-biphényl]-2-ol, 6-amino-3',6' diméthyl [1,1'-biphényl]-2-ol, 6-amino-2',45' triméthyl [1,1'-biphényl]-2-ol, 6-amino-2',3',5' triméthyl [1,1'-biphényl]-2-ol, 6-amino-2',3',6' triméthyl [1,1'-biphényl]-2-ol, 6-amino-4' chlor[1,1'-biphényl]-2-ol, 6-amino-3' chlor[1,1'-biphényl]-2-ol, 6-amino-2' chlor[1,1'-biphényl]-2-ol, 6-amino-4' fluor[1,1'-biphényl]-2-ol, 6-amino-3' fluor[1,1'-biphényl]-2-ol, 6-amino-2' fluor[1,1'-biphényl]-2-ol, 6-amino-4' brom[1,1'-biphényl]-2-ol, 6-amino-3' brom[1,1'-biphényl]-2-ol, 6-amino-2' brom[1,1'-biphényl]-2-ol, 6-amino-3',5' dichlor[1,1'-biphényl]-2-ol, 6-amino-3'5' difluor[1,1'-biphényl]-2-ol, 6-amino-3'-brom-5' méthyl 1,1' biphényl-2-ol, 6-amino-4'-(trifluoro méthyl)-[1,1'-biphényl]-2-ol, 6-amino-3'-(trifluoro méthyl)-[1,1'-biphényl]-2-ol, 6-amino-2'-(trifluoro méthyl)-[1,1'-biphényl]-2-ol, 6-amino-4'-nitro-[1,1'-biphényl]-2-ol, 6-amino-3'-nitro-[1,1'-biphényl]-2-ol, 6-amino-2'-mitro-[1,1'-biphényl]-2-ol, 6-amiono-3'-nitro-[1.1'-biphényl]-2-ol, 6-amino-4'-méthyl-3'-nitro[1.1'-biphényl]-2-ol, 6-amino-2'-méthyl-3'-nitro-[1,1'-biphényl]-2-ol, 6-amino-2'-nitro-4'-(trifluoro méthyl)[1,1'-biphényl]-2-ol, 6-amino-3'-nitro-5'-(trifluoro méthyl)[1,1'-biphényl]-2-ol, 6-amino-3'-nitro-4'-(trifluoro méthyl)[1,1'-biphényl]-2-ol, 6-amino-3'-nitro-2'-(trifluoro méthyl)-[1,1'-biphényl]-2-ol, 6-amino-2'-hydroxy-[1,1'-biphényl]-4-carbone nitrile, 6-amino-2'-hydroxy-[1,1'-biphényl]-3-carbone nitrile, 6-amino-4'-méthoxy-[1,1'-biphényl]-2-ol, 6-amino-34'-méthoxy-[1,1'-biphényl]-2-ol, 6-amino-2'-méthoxy-[1,1'-biphényl]-2-ol, 6-amino-4'-éthoxy-[1,1'-biphényl]-2-ol, 6-amino-3'-éthoxy-[1,1'-biphényl]-2-ol, 6-amino-2'-éthoxy-[1,1'-biphényl]-2-ol, 6-amino-3',4'-diméthoxy-[1,1'-biphényl]-2-ol, 2',4'-diméthoxy-[1,1'-biphényl]-2-ol, 6-amino-23',5'-diméthoxy-[1,1'-biphényl]-2-ol, 36-amino-2-(1,3-benzodioxol-5-yl)-phénol, 6-amino-4'-méthoxy-3'-méthyl-[1,1'-biphényl]-2-ol, 6-amino-4'-méthoxy-2'-nitro-[1,1'-biphényl]-2-ol, 6-amino-4'-méthoxy-32'-nitro-[1,1'-biphényl]-2-ol, 6-amino-4'-phénoxy-[1,1'-biphényl]-2-ol, 6-amino-4'-méthyl thio-[1,1'-biphényl]-2-ol, 6-amino-3'-méthyl thio-[1,1'-biphényl]-2-ol, 6-amino-2'-méthyl thio-[1,1'-biphényl]-2-ol, 6-amino-[1,1'-biphényl]-2,4'-diol, 6-amino [1,1'-biphényl]2,3'-diol, 6-amino [1,1'-biphényl]2,2'-diol, 2,2',3'-trihydroxy-6-amino [1,1'-biphényl], 2,2',4'-trihydroxy-6-amino [1,1'-biphényl], 2,2',5'-trihydroxy-6-amino [1,1'-biphényl], 2,2',6'-trihydroxy-6-amino [1,1'-biphényl, 2,3'4'-trihydroxy-6-amino [1,1'-biphényl], 2,3',5'-trihydroxy-6-amino-[1,1'-biphényl], 6-amino-2'-méthyl [1,1'-biphényl]-2,4'-diol, 2',6-diamino [1,1'-biphényl]-2-ol, 3'6-diamino [1,1'-biphényl]-2-ol, 4'6-diamino [1,1'-biphényl]-2-ol, 4',6-diamino [1,1'-biphényl]-2,2'-diol, 3',6-diamino [1,1'-biphényl]-2,2'-diol, 3',6-diamino [1,1'-biphényl]-2,4'-diol, 3'6-diamino [1,1'-biphényl]-2,5'-diol, 3'6-diamino[1,1'-biphényl]-2,6'-diol, 2',3'6-triamino [1,1'-biphényl]-2-ol, 2',4'6-triamino [1,1'-biphényl]-2-ol, 2',5'6-triamino [1,1'-biphényl]-2-ol, 2',6'6-triamino [1,1'-biphényl]-2-ol, 3',4',6-triamino [1,1'-biphényl]-2-ol, 3',45',6-triamino [1,1'-biphényl]-2-ol, 1-(6'-amino-2'-hydroxy-1,1'-biphényl-4-yl) ethanone, 6-amino-1,1',3',1"-terphényl-2-ol, 6-amino-1,1':4',1"-terphényl-2-ol, 6-amino-4'-(amino méthyl)-1,1'-biphényl-2-ol, 6-amino-3'-(amino méthyl)-1,1'-biphényl-2-ol, 6-amino-2'-(amino méthyl)-1,1'-biphényl-2-ol, (6'-amino-2'-hydroxy-1,1'-biphényl-4-yl) acétone nitrile ; (6'-amino-2'-hydroxy-1,1'-biphényl-3-yl) acétone nitrile, (6'-amino-2'-hydroxy-1,1'-biphényl-2-yl) acétone nitrile, (6'-amino-2'-hydroxy-1,1'-biphényl-2-carbaldehyde, 6'-amino-2'-hydroxy-1,1'-biphényl-3-carbaldéhyde, 6'-amine-2'-hydroxy-1,1'-biphényl-4-carbaldéhyde, 3-amino-2-(1-naphtyl)-phénol, 3-amino-2-(2-naphtyle)-phénol, 3-amino-2-(2,3-dihydro-benzo[1,4]dioxin-6-yl)-phénol, 3-amino-2-(2,3-dihydro-benzo[1,4]dioxin-5-yl)-phénol, 3-amino-2-(2,3-dihydro-benzofuran-5-yl)-phénol, 3-amino-2-(2,3-dihydro-benzofuran-4-yl)-phénol, 3-amino-2-(4-pyridinyl)-phénol, 3-amino-2-(3-pyridinyl)-phénol, 3-amino-2-(2-pyridinyl)-phénol, 3-amino-2-(3-méthyl-2-pyridinyl)-phénol, 3-amino-2-(4-méthyl-2-pyridinyl)-phénol, 3-amino-2-(5-méthyl-2-pyridinyl)-phénol, 3-amino-2-(6-méthyl-2-pyridinyl)-phénol, 3-amino-2-(3-chlor-2-pyridinyl)-phénol, 3-amino-2-(4-chlor-2-pyridinyl)-phénol, 3-amino-2-(5-chlor-2-pyridinyl)-phénol, 3-amino-2-(6-chlor-2-pyridinyl)-phénol, 3-amino-2-(3-fluor-2-pyridinyl)-phénol, 3-amino-2-(4-fluor-2-pyridinyl)-phénol, 3-amino-2-(4-fluor-5-pyridinyl)-phénol, 3-amino-2-(6-fluor-2-pyridinyl)-phénol, 3-amino-2-(3-trifluoro-méthyl-2-pyridinyl)-phénol, 3-amino-2-(4-trifluoro-méthyl-2-pyridinyl)-phénol, 3-amino-2-(5-trifluoro-méthyl-2-pyridinyl)-phénol, 3-amino-2-(6-trifluoro-méthyl-2-pyridinyl)-phénol, 3-amino-2-(3-nitro-2-pyridinyl)-phénol, 3-amino-2-(4-nitro-2-pyridinyl)-phénol, 3-amino-2-(5-nitro-2-pyridinyl)-phénol, 3-amino-2-(6-nitro-2-pyridinyl)-phénol, 3-amino-2-(2-méthyl-3-pyridinyl)-phénol, 3-amino-2-(42-méthyl-3-pyridinyl)-phénol, 3-amino-2-(5-méthyl-3-pyridinyl)-phénol, 3-amino-2-(6-méthyl-3-pyridinyl)-phénol, 3-amino-2-(2-chlor-3-pyridinyl)-phénol, 3-amino-2-(4-chlor-3-pyridinyl)-phénol, 3-amino-2-(5-chlor-3-pyridinyl)-phénol, 3-amino-2-(64-chlor-3-pyridinyl)-phénol, 3-amino-2-(2-brom-3-pyridinyl)-phénol, 3-amino-2-(4-brom-3-pyridinyl)-phénol, 3-amino-2-(5-brom-3-pyridinyl)-phénol, 3-amino-2-(6-brom-3-pyridinyl)-phénol, 3-amino-2-(2-nitro-3-pyridinyl)-phénol, 3-amino-2-(4-nitro-3-pyridinyl)-phénol, 3-amino-2-(5-nitro-3-pyridinyl)-phénol, 3-amino-2-(6-nitro-3-pyridinyl)-phénol, 3-amino-2-(5-pyrimidinyl)-phénol et 3-amino-2-(4-pyrimidinyl)-phénol ainsi que leurs sels hydrosolubles physiologiquement compatibles.

3. Dérivé de 3-amino phénol selon la revendication 1, **caractérisé en ce que** dans la formule (I) : (i) **R1** est égal à un groupe de formule (II) avec **R2** ou à **R6** égal à l'hydrogène, ou (ii) **R1** est égal à un groupe de formule (III) avec **X1** ou à **X5** égal à **C-R7** ou **C-R11,** respectivement, dans laquelle **R7** ou **R11,** respectivement, est égal à l'hydrogène.

4. Dérivé de 3-amino phénol selon l'une des revendications 1 à 3, **caractérisé en ce qu'**il est choisi parmi [le groupe constitué de] 6-amino-3'-méthoxy-[1,1'-biphényl]2-ol, 6-amino-[1,1'-biphényl]2,4'-diol, 3-amino-2-(2,3-dihydro-benzo[1,4]dioxin-6-yl)-phénol, 3-amino-2-(2,3-dihydro-benzofuran-5-yl)-phénol, 3-amino-2-(3-pyridinyl)-phénol, 3-amino-2-(4-pyridinyl)-phénol et 3-amino-2-(5-pyrimidinyl)-phénol ainsi que leurs sels hydrosolubles physiologiquement compatibles.

5. Agent pour colorer des fibres de kératine sur la base d'une association substance de développeur/substance de copulant, **caractérisé en ce qu'**en tant que substance de copulant il contient au moins un dérivé de 3-amino phénol de formule (I) selon l'une des revendications 1 à 4.

6. Agent selon la revendication 5, **caractérisé en ce que** le dérivé de 3-amino phénol de formule (I) est contenu en une quantité allant de 0,005 à 20 pour cent en poids.

7. Agent selon la revendication 5 ou 6, **caractérisé en ce que** la substance de développeur est choisie parmi le groupe constitué de 1,4-diamino-benzène, 1,4-diamino-2-méthyl benzène, 1,4-diamino-2,6-diméthyl benzène, 1,4-diamino-3,5-diéthyl benzène, 1,4-diamino-2,5-diméthyl benzène, 1,4-diamino-2,3-diméthyl benzène, 2-chlor-1,4-diamino benzène, 1,4-(diamino-2-(2-thiényl) benzène, 1,4-(diamino-2-(3-thiényl) benzène, 1,4-diamino-2-(pyridine-3-yl) benzène, 2,5-diamino-biphényl, 1,4-diamino-2-méthoxy méthyl benzène, 1,4-diamino-2-(2-hydroxy éthoxy) benzène, 2-(2-(acétyle amino) éthoxy)-1,4-diamino benzène, 4-phényl amino aniline, 4-diméthyl amino aniline, 4-diéthyl amino aniline, 4-dipropyl amino aniline, 4-[éthyl (2-hydroxy éthyl) amino] aniline, 4-[éthyl (2-hydroxy éthyl) amino] aniline, 4-[éthyl di(2-hydroxy éthyl) amino] aniline, 4-[di(2-hydroxy éthyl) amino]-2-méthyl aniline, 4-[(2-méthoxy éthyl) amino] aniline, 4-[(2-méthoxy éthyl) amino] aniline, 4-[3-hydroxy propyl) amino] aniline, 4-[2,3-dihydroxy propyl) amino] aniline, 1,4-diamino-2-(1-hydroxy éthyl) benzène, 1,4-diamino-2-(21-hydroxy éthyl) benzène, 1,4-diamino-2-(1-méthyl éthyl) benzène, 1,3-bis[(4-amino phényl)(2-hydroxy éthyl)amino]-2-propanol, 1,4-bis[(4-amino phényl)amino]-butane, 1,8-bis(2,5-diamino phénoxy)-3,6-dioxaoctane, 4-amino phénol, 4-amino-3-méthyl phénol, 4-amino-3-(hydroxy méthyl) phénol, 4-amino-3-fluor-phénol, 4-méthyl amino phénol, 4-amino-2-(amino méthyl) phénol, 4-amino-2-(hydroxy méthyl) phénol, 4-amino-2-fluor phénol, 4-amino-2[(2-hydroxy éthyl) amino] méthyl phénol, 4-amino-2-méthyl phénol, 4-amino-2-(méthoxy méthyl) phénol, 4-amino-2-(2-hydroxy éthyl) phénol, acide 5-amino salicylique, 2,5-diamino pyridine, 2,4,5,6-tétra-amino pyrimidine, 2,5,6-triamino-4-(1H) pyrimidone, 4,5-diamino-méthyl phényl) méthyl]-1H pyrazole, 1-[(4-chlor phényl) méthyl]-4,5-diamino-1H-pyrazole, 4,5-diamino-1-méthyl-1H-pyrazole, 2-amino phénol, 2-amino-6-méthyl phénol, 2-amino-5-méthyl phénol et 1,2,4-trihydroxy benzène.

8. Agent selon l'une des revendications 5 à 7, **caractérisé en ce qu'**il contient, en plus des composés de formule (I) au moins une autre substance de copulant connue qui est choisie parmi le groupe constitué de N-(3-diméthyl amino phényl) carbamide, 2,6-diamino pyridine, 2-amino-4-[2-hydroxy éthyl) amino] anisole, 2,4-diamino-1-fluor-5-méthyl benzène, 2,4-diamino-1-méthoxy-5-méthyl benzène, 2,4-diamino-1-éthoxy-5-méthyl benzène, 2,4-diamino-1-(2-hydroxy éthoxy)-5-méthyl benzène, 2,4-di[(2-hydroxy éthyl) amino]-1,5-diméthoxy-benzène, 2,3-diamino-6-méthoxy pyridine, 3-amino-6-méthoxy-2-(méthyl amino) pyridine, 2,6-diamino-3,5-diméthoxy pyridine, 3,5-diamino-2,6-diméthoxy pyridine, 1,3-diamino benzène, 2,4-diamino-1-(2-hydroxy éthoxy) benzène, 1,3-diamino-4-(2,3-dihydroxy propoxy) benzène, 1,3-diamino-4-(3-hydroxy propoxy) benzène, 1,3-diamino-4-(2-méthoxy éthoxy) benzène, 2,4-diamino-1,5-di(2-hydroxy éthoxy) benzène, 1-(2-amino éthoxy)-2,4-diamino benzène, 2-amino-1-(2-hydroxy éthoxy)-4-méthyl amino benzène, acide 2,4-diamino phénoxy acétique, 3-[voir original pour diagramme](2-hydroxy éthyl) amino] aniline, 4-amino-2-di[2-hydroxy éthyl) amino]-1 éthoxy benzène, 5-méthyl-2-(1-méthyl éthyl) phénol, 3-[(2-hydroxy éthyl) amino] aniline, 3-[(2-amino éthyl) amino] aniline, 1,3-di(2,4-diamino phénoxy) propane, di(2,4-diamino phénoxy) méthane, 1,3-diamino-2,4-diméthoxy benzène, 2,6-bis(2-hydroxy éthyl) amino toluol, 4-hydroxy indole, 3-diméthyl amino phénol, 3-diéthyl amino phénol, 5-amino-2-méthyl phénol, 5-amino-4-fluor-2-méthyl phénol, 5-amino-4-méthoxy-2-méthyl phénol, 5-amino-4-éthoxy-2-méthyl phénol, 3-amino-2,4-dichlor phénol, 5-amino-2,4-dichlor phénol, 3-amino phénol, 2-[(3-hydroxy phényl) amino] acétamide, 5-[(2-hydroxy amino]-2-méthyl phénol, 5-amino-2-éthyl phénol, 5-amino-2-méthoxy phénol, 2-(4-amino-2-hydroxy phénoxy) ethanol, 5-[(3-hydroxy propyl) amino]-2-méthyl phéneol, 3-[(2,3-dihydroxy propyl) amino]-2-méthyl phénol, 3-[(2-hydroxy éthyl) amino]-2-méthyl phénol, 2-amino-3-hydroxy pyridine, 2,6-dihydroxy-3,4-diméthyl pyridine, 5-amino-4-chlor-2-méthyl phénol, 1-naphtol, 2-méthyl-1-naphtol, 1,5-dihydroxy naphtaline, 1,7-dihydroxy naphtaline, 2,3-dihydroxy naphtaline, 2,7-dihydroxy naphtaline, acétate de 2-méthyl-1-naphtol, 1,3-dihydroxy benzène, 1-chlor-2,4-dihydroxy benzène, 2-chlor-1,3-dihydroxy benzène, 1,2-dichlor-3,5-dihydroxy-4-méthyl benzène, 1,5-dichlor-2,4-dihydroxy benzène, 1,3-dihydroxy-2-méthyl benzène, 3,4-méthylene dioxy phénol, 3,4-méthylene dioxy aniline, 5-[(2-hydroxy éthyl) amino]-1,3-benzodioxole, 6-brom-1-hydroxy-3,4-méthylene dioxy benzène, acide 3,4-diamino benzoïque, 3,4-dihydro-6-hydroxy-1,4(2H)-benzoxacine, 6-amino-3,4-dihydro-1,4(2H)-benzoxacine, 3-méthyl-1-phényl-5-pyrazolone, 5,6-dihydroxy indole, 5,6-dihydroxy indoline, 5-hydroxy indole, 6-hydroxy indole, 7-hydroxy indole et 2,3 indolindione.

9. Agent selon l'une des revendications 5 à 8, **caractérisé en ce que** les substances de développeur et les substances de copulant, sur base de la quantité globale du colorant, sont dans chaque cas contenues en une quantité globale allant de 0,005 à 20 pour cent en poids.

10. Agent selon l'une des revendications 5 à 9, **caractérisé en ce qu'**il contient également au moins une teinture à vision directe.

11. Agent selon l'une des revendications 5 à 10, **caractérisé en ce qu'**il a une valeur de pH allant de 6,5 à 11,5.

12. Agent prêt à l'emploi pour la coloration oxydative de fibres de kératine, qui dans un milieu approprié pour la coloration contient au moins une substance de développeur et au moins une substance de copulant ainsi qu'au moins un agent d'oxydation, **caractérisé en ce qu'**il contient en tant que substance de copulant au moins un dérivé de 3-amino phénol de formule (I) selon l'une des revendications 1 à 4.

13. Agent selon l'une des revendications 5 à 12, **caractérisé en ce que** c'est un agent de coloration capillaire.

14. Procédé pour la coloration oxydative de cheveux, en particulier de cheveux humains, **caractérisé en ce qu'**avant application, un agent de coloration capillaire selon l'une des revendications 5 à 13 est mélangé avec un agent d'oxydation puis est appliqué sur les cheveux et laissé au repos pour agir pendant 45 minutes à une température de 15 à 50 °C ; les cheveux sont ensuite rincés avec de l'eau et, facultativement, shampouinés, puis séchés.
